# FASCICULE DE BREVET EUROPEEN

(11) **EP 0 777 734 B1**
(45) Date de publication et mention de la délivrance du brevet: **08.03.2006**
(21) Numéro de dépôt: 95928541.2
(22) Date de dépôt: 24.08.1995
(51) Int. Cl.: C12N 15/32, C07K 14/325, C12Q 1/68, A01N 63/00, C07K 16/12, C12N 1/21, C12R 1/07

(54) **NOUVEAUX POLYPEPTIDES A ACTIVITE TOXIQUE VIS-A-VIS D'INSECTES DE LA FAMILLE DES DIPTERES**
NEUE POLYPEPTIDE MIT TOXISCHER AKTIVITÄT GEGEN INSEKTEN DER FAMILIE DER DIPTEREN
NEW POLYPETIDES HAVING A TOXIC ACTIVITY AGAINST INSECTS OF THE DIPTER FAMILY

(30) Priorité: 25.08.1994 FR 9410299
(43) Date de publication de la demande: 11.06.1997
(73) Titulaire: INSTITUT PASTEUR, 75015 Paris (FR)
(72) Inventeur: DELECLUSE, Armelle, F-60520 Thiers-sur-Theve (FR); THIERY, Isabelle, F-92320 Chatillon (FR)
(74) Mandataire: Desaix, Anne
(86) Numéro de dépôt international: PCT/FR1995/001116
(87) Numéro de publication internationale: WO 1996/006171

(56) Documents cités:
- EP-A- 0 216 481
- EP-A- 0 480 762
- WO-A-89/07605
- APPL ENVIRON MICROBIOL 57 (4). 1991. 1075-1081, YU, Y-M ET AL. 'CHARACTERIZATION OF MOSQUITOCIDAL ACTIVITY OF BACILLUS- THURINGIENSIS -SSP-FUKUOKAENSIS CRYSTAL PROTEINS.'
- SOUTHEAST ASIAN J TROP MED PUBLIC HEALTH 21 (2). 1990. 281-288, LEE, H. & SELEENA, P. 'ISOLATION OF INDIGENOUS LARVICIDAL MICROBIAL CONTROL AGENTS OF MOSQUITOS: THE MALAYSIAN EXPERIENCE.'
- APPLIED AND ENVIRONMENTAL MICROBIOLOGY 61 (8). 2965-2969, Août 1995 XP 000560332 KAWALEK, M. ET AL. 'Isolation and identification of novel toxins from a new mosquitocidal isolate from Malaysia, Bacillus thuringiensis subsp. jegathesan'
- APPL.ENVIRON.MICROBIOL.; 61 (12), 4230-35, Décembre 1995 XP 000560331 DELECLUSE, A. ET AL. 'Cloning and expression of a novel toxin gene from Bacillus thuringiensis subsp. jegathesan encoding a highly mosquitocidal protein;crystal protein expression in Escherichia coli and non-toxic strain for improved insect biological control agent production'

## Description

La lutte biologique contre les insectes de la famille des Diptères, qui comprend par exemple les moustiques et les Simulies vecteurs de maladies tropicales est effectuée principalement à l'aide de la bactérie Bacillus thuringiensis ser. israelensis (Bti) du sérotype H14 ou à l'aide de Bacillus sphaericus. Ces deux bactéries synthétisent pendant la sporulation des protéines assemblées sous forme de cristaux, toxiques par ingestion pour les larves d'insectes. Les cristaux de B thuringiensis ser. israelensis sont composés de 4 polypeptides majeurs CryIV A (125 kDa), CryIV B (135 kDa), CryIV D (68 kDa) et Cyt A (28 kDa), (Höfte H et al., 1989 Microbiol. Rev. 53: 242-255). Les cristaux de B. sphaericus sont constitués par 2 polypeptides de 51 et 42 kDa. Ces protéines ont des spécificités différentes et chacune d'entré elles contribue à la toxicité totale, agissant le cas échéant en synergie. Dans le but de pallier l'apparition éventuelle d'insectes résistants aux toxines de Bti, la recherche de nouvelles souches présentant une activité sur les moustiques a été entreprise.

Les souches de B thuringiensis actives sur moustiques peuvent être classées en quatre groupes selon leur activité larvicide, leur composition en protéines du cristal et la présence de gènes apparentés à ceux de la souche B thuringiensis ser. israelensis :
(1) le groupe 1 contient les six souches de B thuringiensis désignées respectivement par morrisoni PG14 (H8a8b), canadensis 11S2-1 (H5a5c), thompsoni B175 (H12), malaysiensis IMR81.1 (H36), K6 (autoagglutinante), et B51 (autoagglutinante) ayant une activité larvicide similaire et des polypeptides du cristal semblables à ceux de B thuringiensis ser. israelensis,
(2) le groupe 2 inclut les deux souches de B thuringiensis medellin 163-131 (H30) et jegathesan 367 (H28a28c) qui sont presque aussi toxiques que la souche B thuringiensis ser. israelensis mais qui produisent des polypeptides différents,
(3) le groupe 3 comprend la souche darmstadiensis 73E10-2 (H10a10b) qui synthétise des polypeptides différents que ceux trouvés dans les cristaux de B thuringiensis ser. israelensis mais qui est active sur une espèce seulement de moustiques, et
(4) le groupe 4 qui inclut les deux souches fukuokaensis (H3a3d3e) et Kyushuensis 74 F6-18 (H11a11c) qui sont seulement faiblement toxiques.

Etant donné la faible toxicité des souches des groupes 3 et 4, ces souches n'ont pas été étudiées de façon approfondie.

Parmi toutes les souches isolées, la souche de Bacillus thuringiensis ser. jegathesan 367 (Btjeg), du sérotype H28a 28c, paraît intéressante tant du point de vue de son activité que de la composition polypeptidique de ses cristaux. Cette bactérie produit comme Bti au cours de la sporulation des cristaux toxiques par ingestion pour les larves de moustiques. Cette souche a été isolée en Malaisie par L. LEE et identifiée comme appartenant à un nouveau sous-type.

Les cristaux de B thuringiensis ser. jegathesan contiennent 7 polypeptides majeurs ayant un poids moléculaire de 80, 72-70, 65, 37, 26 et 16 kDa respectivement. La protéine de 37 kDa est immunologiquement apparentée à un composant du cristal de B thuringiensis ser. israelensis alors que les autres protéines donnent seulement des réactions croisées faibles et variables. Aucun gène apparenté à ceux de B thuringiensis ser. israelensis n'a été détecté dans cette souche, indiquant que les protéines du cristal pourraient être codées par une nouvelle classe de gènes de toxines.

Les inventeurs ont identifié au sein de l'ADN total d'une souche Btjeg 367, des séquences codant pour des polypeptides susceptibles d'induire et/ou de participer à l'activité toxique de la souche vis-à-vis d'insectes de la famille des Diptères en particulier.

L'invention a donc pour objet des séquences de nucléotides codant pour des polypeptides à activité toxique vis-à-vis d'insectes. Des insectes cibles sont par exemple des insectes de la famille des Diptères, notamment des moustiques ou des Simulies, et en particulier les larves de ces insectes. Cependant, il n'est pas exclu que les polypeptides obtenus à partir des séquences de l'invention puissent présenter une activité vis-à-vis d'insectes d'autres familles.

La demande concerne également des polypeptides ayant une activité larvicide vis-à-vis d'insectes, ou des polypeptides capables de participer à une telle activité toxique, le cas échéant en agissant en synergie avec des polypeptides déterminant cette activité.

Ainsi les polypeptides de l'invention sont susceptibles soit d'induire l'activité toxique, soit d'augmenter le niveau de la toxicité, vis-à-vis d'une cible donnée.

Entrent également dans le cadre de l'invention, des compositions larvicides comprenant à titre de principe actif les polypeptides de l'invention ou des organismes recombinants capables d'exprimer de tels polypeptides, le cas échéant associés à d'autres constituants, par exemple d'autres polypeptides ou cellules recombinantes capables d'augmenter l'activité toxique recherchée, le cas échéant issus d'autres organismes, par exemple Bacillus thuringiensis, Bacillus sphaericus, Clostridium bifermentans.

Un premier groupe de séquences contient une première séquence de nucléotides caractérisée en ce qu'elle correspond au fragment HindIII d'environ 4,3 kb pouvant être obtenu à partir du plasmide pJEG80.1 déposé à la CNCM sous le numéro I-1469, le 23 août 1994 ou pouvant hybrider dans des conditions stringentes avec ce plasmide.

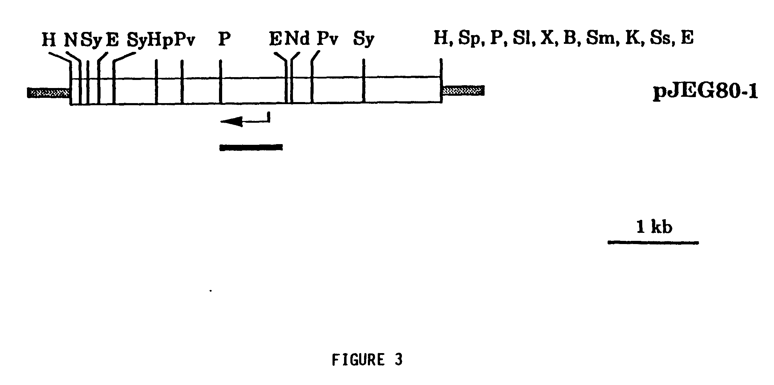

La carte de restriction de la séquence de Btjeg contenue dans le plasmide recombinant pJEG80.1 est représentée à la figure 3 et montre la position du début du gène jeg80, ainsi que son sens de transcription.

Selon un mode de réalisation particulier de la présente invention, une séquence de nucléotides de ce premier groupe est comprise dans le fragment HindIII d'environ 4,3 kb représenté à la figure 3, tel qu'il peut être isolé à partir du plasmide pJEG80.1 déposé à la CNCM. Un fragment intéressant est par exemple le fragment HindIII-NdeI d'environ 2,2 kb. Ce fragment contient l'origine du gène jeg80.

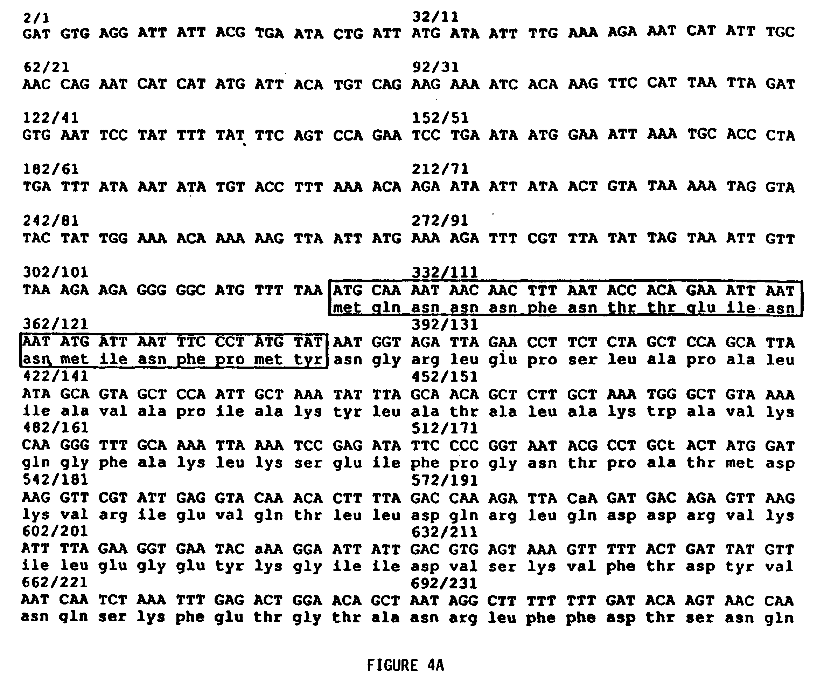

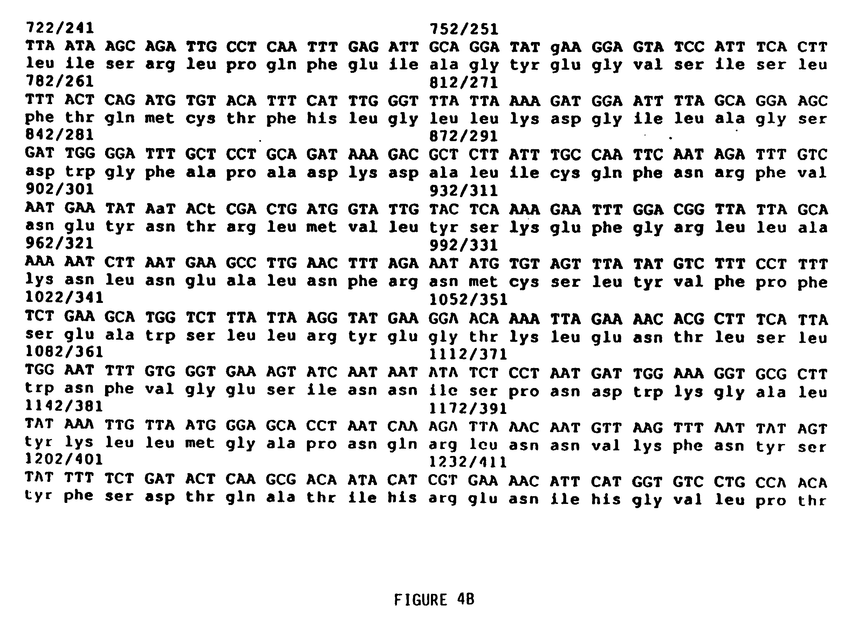

Selon un autre mode de réalisation particulier de l'invention, la séquence de nucléotides répondant à la définition précédente est en outre caractérisée en ce qu'elle comprend l'enchaînement de nucléotides représenté à la figure 4.

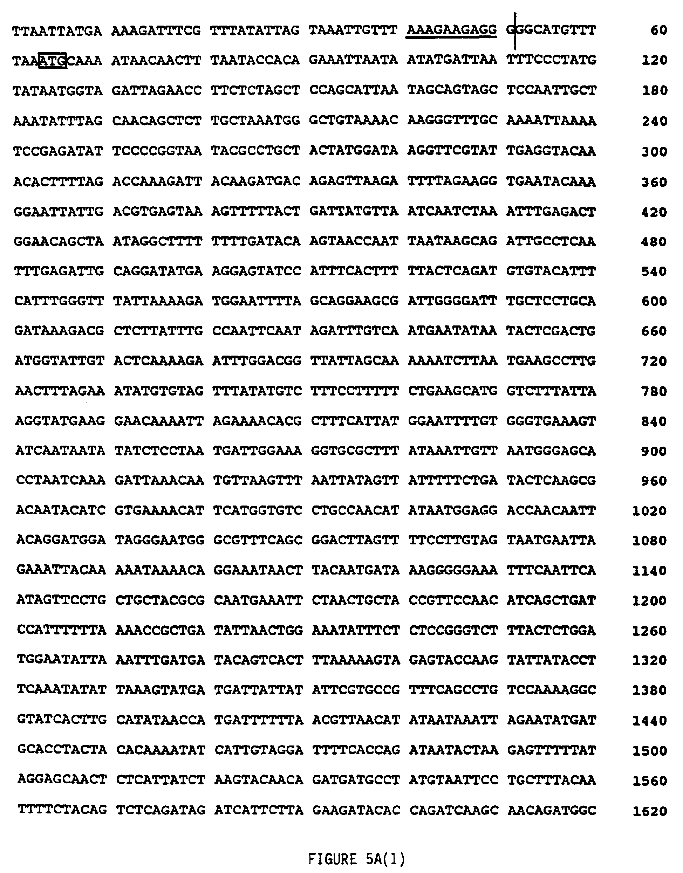

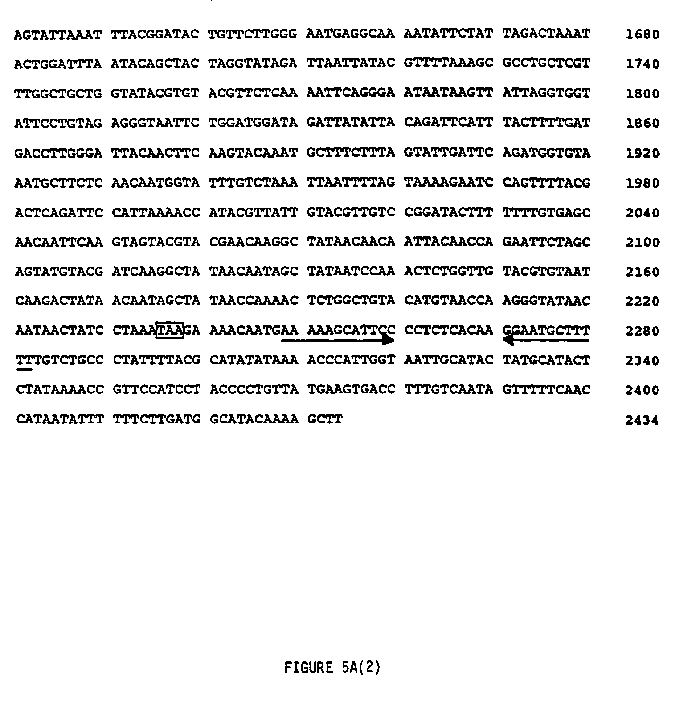

L'invention concerne aussi le gène jeg80 représenté à la figure 5A dont la séquence codante est comprise entre les nucléotides 64 et 2238.

L'invention vise aussi la séquence non codante en amont de la séquence codante du gène jeg80, qui contient les séquences régulatrices de l'expression du gène. En particulier, l'invention vise le fragment compris entre les nucléotides 1 et 124 de l'enchaînement représenté à la figure 4.

L'invention concerne également des séquences de nucléotides modifiées par rapport aux séquences précédemment définies, par exemple par délétion, addition ou substitution de nucléotides, caractérisées en ce qu'elles hybrident dans des conditions stringentes avec une des séquences précédemment identifiées, et en ce qu'elles codent en outre pour un polypeptide ayant une activité toxique vis-à-vis d'insectes de la famille des Diptères ou participant à cette activité.

On entend par polypeptides selon la présente description, des peptides, polypeptides ou protéines, ou toute séquence d'acides aminés ayant les propriétés requises.

L'activité constatée de toxicité vis-à-vis d'insectes de la famille des Diptères ne doit en aucun cas être considérée comme limitative pour la définition des séquences de l'invention. Au contraire, cette référence aux Diptères constitue uniquement un critère d'évaluation de l'intérêt d'une séquence donnée, sachant toutefois que les insectes cibles, s'agissant de l'activité toxique, peuvent appartenir à d'autres familles.

L'activité toxique évaluée par rapport à des insectes de la famille des Diptères peut par exemple être testée chez des moustiques ou des Simulies ou chez les larves de ces insectes.

L'activité toxique du produit d'expression d'une séquence de nucléotides de l'invention peut être évaluée en mesurant la dose létale nécessaire pour tuer 50% d'un échantillon de larves d'insectes testées (LC₅₀), lorsque le test est réalisé dans 150 ml d'eau avec 25 larves par gobelet, ces larves étant de stade L4 pour Aedes aegypti et Culex pipiens et de stade L3 pour Anopheles stephensi. Des quantités variables de toxines sont ajoutées dans les gobelets. Les larves de Culex et d'Anophèles sont nourries à la levure de bière, à raison de 50 mg/1. La lecture du test est faite à 24 h et 48 h.

Une séquence de nucléotides codant pour un polypeptide ayant une activité toxique vis-à-vis d'insectes de la famille des Diptères selon l'invention est par exemple une séquence codant pour un polypeptide ayant un poids moléculaire d'environ 80 kDa.

L'invention a également pour objet tout fragment issu d'une séquence de nucléotides répondant à l'un des critères précédents et hybridant dans des conditions stringentes avec l'une de ces séquences, ce fragment ayant au moins 9 nucléotides. De tels fragments sont notamment destinés à être utilisés en tant que sondes d'hybridation ou en tant qu'oligonucléotides amorces pour la réalisation de réactions d'amplification en chaîne telles que la PCR. A titre d'exemple, une séquence de nucléotides intéressante est la séquence j80, répondant à l'enchaînement suivant :

La présente demande a également pour objet un deuxième groupe de séquences de nucléotides dont un représentant est par exemple une séquence de nucléotides caractérisée en ce qu'elle code pour un polypeptide qui, en association avec un polypeptide codé par une séquence nucléotidique du premier groupe présentée plus haut, est capable de participer à l'activité toxique de ce dernier vis-à-vis d'insectes de la famille des Diptères, cette séquence hybridant en outre avec l'oligonucléotide j66 ayant la séquence suivante :

La définition de cette séquence nucléotidique par rapport à sa capacité de participer à l'activité toxique du polypeptide codé par l'une des séquences du premier groupe, n'exclut pas la possibilité pour cette séquence nucléotidique de coder pour un peptide ayant de façon isolée une activité toxique vis-à-vis d'insectes de la famille des Diptères.

L'intérét de ce deuxième groupe de séquences nucléotidiques peut par exemple résulter de ce que son association avec des polypeptides codés par le premier groupe de séquences précédemment défini peut conduire à une synergie telle que l'activité toxique de l'association de polypeptides est supérieure à la somme des activités individuelles de chacun des polypeptides de l'association. Une séquence de nucléotides particulière de ce deuxième groupe code pour un polypeptide d'environ 66 kDa.

Un troisième groupe de séquences nucléotidiques est caractérisé en ce qu'elles codent pour des polypeptides qui, en association avec un polypeptide codé par une séquence de nucléotides du premier groupe ou du deuxième groupe, sont capables de participer à l'activité toxique de ces polypeptides vis-à-vis d'insectes de la famille des Diptères, ces séquences nucléotidiques étant en outre caractérisées en ce qu'elles hybrident avec l'oligonucléotide j37 ayant la séquence suivante :

Une séquence nucléotidique préférée de ce troisième groupe est caractérisée en ce qu'elle code pour un polypeptide d'environ 37 kDa.

L'invention a pour objet un quatrième groupe de séquences nucléotidiques, codant pour un polypeptide qui, en association avec l'un au moins des précédents, est également capable de participer à l'activité toxique observée vis-à-vis d'insectes de la famille des Diptères, les séquences de ce quatrième groupe codant pour un polypeptide ayant un poids moléculaire d'environ 70 kDa ou produisant une réaction immunologique avec ce peptide.

Les conditions d'hybridation avec les oligonucléotides sont des conditions d'hybridation stringentes décrites dans le kit "ECL 3' oligo-labelling detection kit" de Amersham, la température d'hybridation étant de 42°C et le deuxième lavage après hybridation étant fait dans 1xSSC - 0,1%SDS.

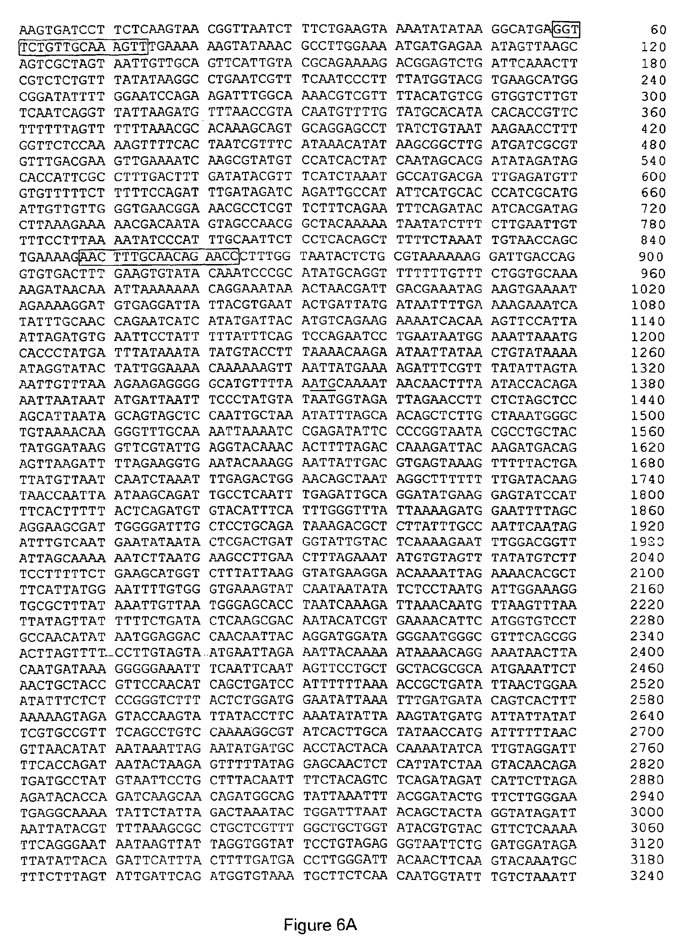

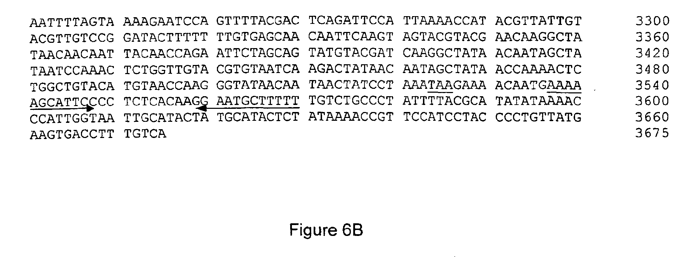

L'invention a aussi pour objet les séquences entourant celle du gène jeg80, correspondant à l'ISjeg entre les nucléotides 2812 et 3618 de la séquence de la figure 6 et de la région intergénique entre les nucléotides 1604 et 284 de la figure 6.

L'invention a également pour objet un vecteur de clonage ou d'expression, caractérisé en ce qu'il comprend une séquence de nucléotides répondant aux définitions précédemment données.

Un vecteur particulièrement préféré est le plasmide pJEG80.1 déposé à la CNCM le 23 août 1994 sous le n° I-1469.

La présente demande a également pour objet des polypeptides caractérisés en ce qu'ils constituent le produit d'expression dans une cellule recombinante, de l'une au moins des séquences de nucléotides du premier, deuxième, troisième ou quatrième groupes telles qu'elles ont été définies dans les pages précédentes.

En particulier l'invention concerne le polypeptide Jeg80 codé par le gène jeg80 représenté à la figure 5A.

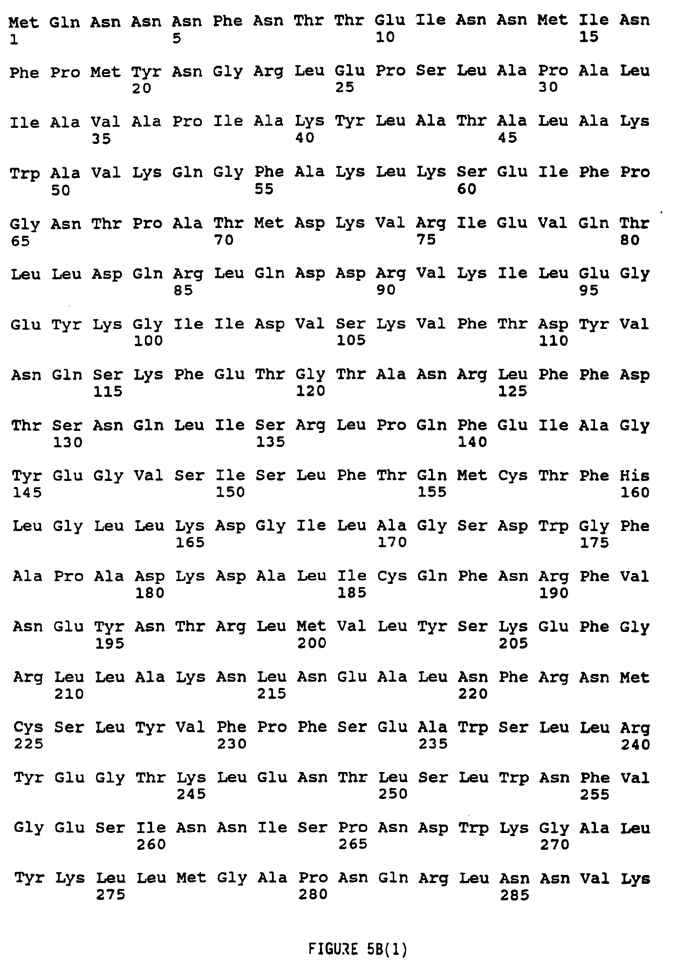

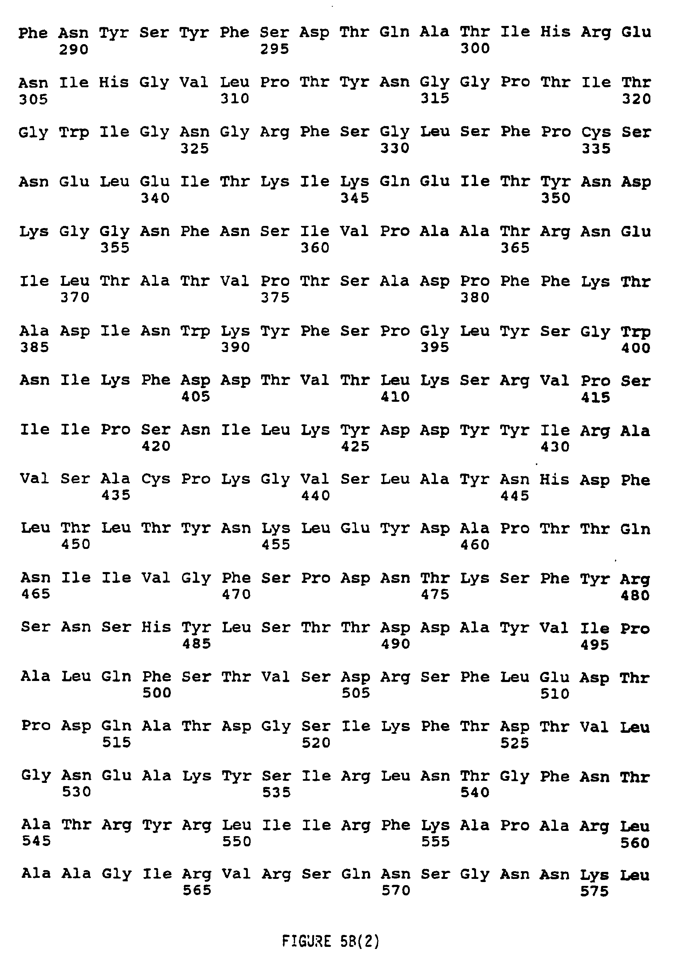

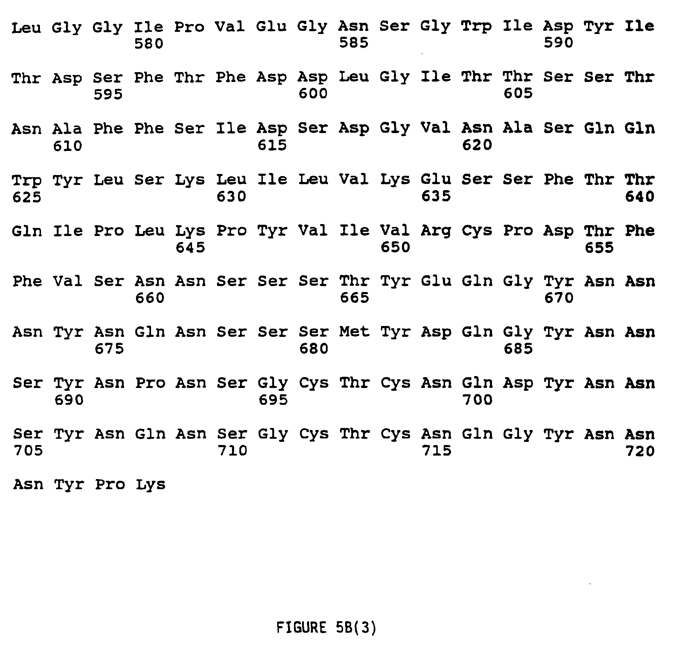

Des polypeptides préférés sont par exemple le polypeptide d'environ 80 kDa comprenant la séquence d'acides aminés représentée à la figure 4, ou le polypeptide d'environ 80 kDa répondant à la séquence d'acides aminés représentée à la figure 5B, ou tout fragment de ce polypeptide réagissant avec des anticorps dirigés contre la protéine de 80 kDa répondant à la séquence de la figure 5B et/ou présentant une activité toxique vis-à-vis de larves d'insectes de la famille des Diptères.

Les polypeptides de l'invention peuvent être utilisés seuls ou en combinaison. Ces combinaisons (ou mélanges) peuvent comprendre différents polypeptides des groupes I, II, III ou IV tels qu'ils ont été décrits ci-dessus et/ou un mélange d'un ou plusieurs de ces polypeptides avec d'autres polypeptides issus d'organismes différents et en particulier de souches de Bti, de B. sphaericus ou de C. bifermentans ayant également une activité toxique vis-à-vis d'insectes.

Entrent également dans le cadre de la présente demande, des cellules recombinantes modifiées par une séquence de nucléotides telles que définies dans les pages précédentes ou par un vecteur contenant une de ces séquences.

Ces cellules hôtes recombinantes sont des cellules procaryotes ou eucaryotes, et il peut s'agir par exemple de cellules de bactéries, par exemple de souches de Bacillus thuringiensis souche Bti ou de Bacillus sphaericus, voire de clostridium bifermantans.

S'agissant de B. thuringiensis, on fera référence à la publication de Lereclus D. t al. (1989), FEMS Microbiology Letters 60, 211-218, dans laquelle la technique de transformation (par introduction des gènes de toxines dans Bt) de B. thuringiensis est décrite.

S'agissant de B. sphaericus, on se reportera par exemple à la publication de Taylor L.D. et al (1990) FEMS Microbiology Letters 66, 125-128.

Une autre cellule selon l'invention peut être une cellule eucaryote, par exemple une cellule végétale.

Les cellules recombinantes peuvent être utilisées pour produire les polypeptides de l'invention, mais peuvent également être utilisées dans des compositions toxiques vis-à-vis d'insectes.

La demande a également pour objet des anticorps polyclonaux dirigés contre l'un des polypeptides ci-dessus définis, ou encore un sérum polyclonal dirigé contre un mélange de plusieurs d'entre eux.

D'autres caractéristiques et avantages de l'invention apparaissent dans les exemples et les figures qui suivent.

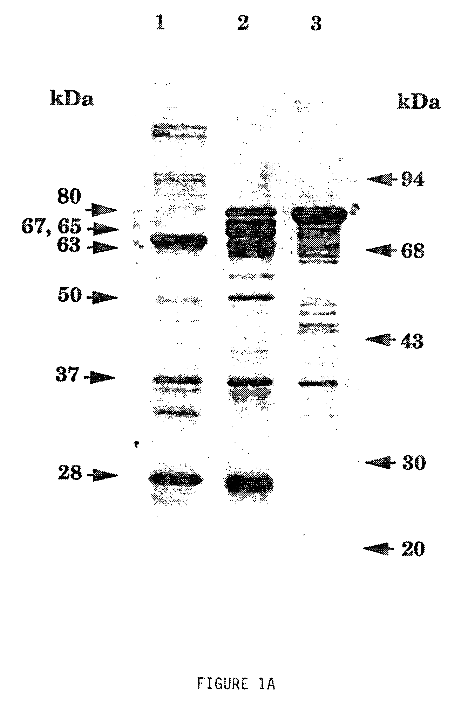

- **Figure 1A -**: composition polypeptidique des cristaux de la souche Btjeg
Les cristaux des souches naturelles Bti et Btjeg, et de la souche recombinante 407 (pJEG80.1) ont été purifiés et déposés sur gel de polyacrylamide 10 %-SDS. Après électrophorèse, le gel a été coloré au bleu de Coomassie. La masse moléculaire (en kDa) de protéines standards est représentée à droite. Les masses moléculaires des protéines de Btjeg sont indiquées à gauche. Puits : 1, Bti ; 2, Btjeg ; 3, 407 (pJEG80.1).

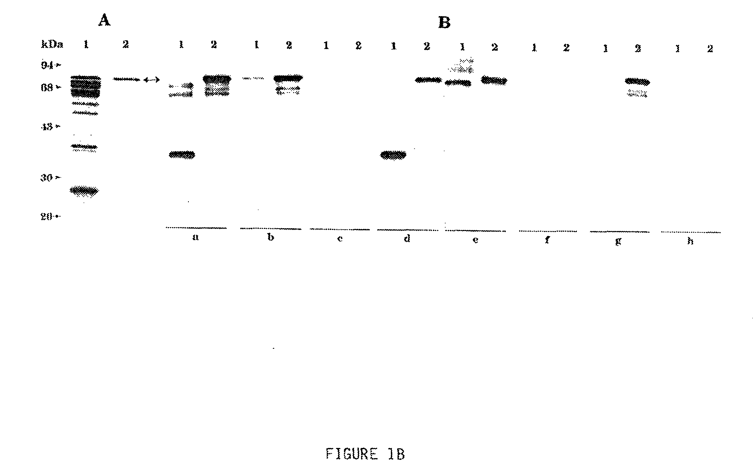

- **Figure 1B -**: **Analyse des protéines contenues dans les inclusions produites par la souche 407 (pJEG80.1)**
**A:** Inclusions purifiées correspondant à 10 µg de protéines soumises à une électrophorèse et colorées au Bleu de Coomassie.
**B:** Inclusions purifiées correspondant à 1 µg de protéine soumise à électrophorèse et transférées sur filtre de nitrocellulose. Le filtre a été incubé avec l'antisérum (dilué 5.000 fois) contre les cristaux totaux de Bt ser. jegathesan (a), ser. medellin (b), ser. darmstadiensis (c), ser. israelensis (d) ou contre les inclusions purifiées solubilisées composées de CryIVA (e), CryIVB (f), CryIVD (g) ou CytA (h). les polypeptides immunoréactifs ont été révélés avec un second anticorps conjugué à la péroxydase (dilué 20.000 fois). Les flèches entre A et B donnent la position de Jeg80. Les nombres à gauche donnent les poids moléculaires (kDa) des protéines standards ; ligne 1 : les inclusions purifiées de la souche Btjeg 407 : ligne 2 : les inclusions purifiées de la souche 407 (pJEG80.1).
- **Figure 2 -**: Sondes utilisées pour déterminer la présence des gènes de toxines de Bti chez Btjeg
- **Figure 3 -**: Carte de restriction du plasmide PJEG80.1 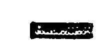 Vecteur pHT315 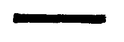 Fragment hybridant avec l'oligonucléotide j80 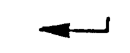 Début du gène jeg80 et sens de transcription 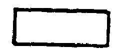 Fragment d'ADN de Btjeg cloné au site HindIII du plasmide pHT315
Site de restriction du polylinker
Aucun site de restriction n'a été trouvé pour les enzymes suivants : BamHI, BgIII, Clal, EcoRV, Kpnl, Ncol, SacI, SacII, SmaI, SphI, XbaI et XhoI H = HindIII ; E = EcoRI ; K = KpnI ; Hp = HpaI ; B = BamHI ; N = NsiI ; Nd = NdeI ; P = PstI ; Pv = PvuII ; Sm = SmaI ; Sp = SphI ; Ss = SstI ; SI = SalI ; Sy = StyI ; X = XbaI.
- **Figure 4 -**: Séquence nucléotidique d'une partie du gène jeg80 et séquence en acides aminés correspondant à la séquence codante : la séquence encadrée représente la séquence en acides aminés déterminée par microséquençage.
- **Figure 5 -**: **A:** Gène jeg80. Le site potentiel de liaison au ribosome est souligné. les codons de départ et d'arrêt de traduction sont indiqués. Les séquences inversées répétées sont marquées par des flèches.
**B:** Séquence d'acides aminés de la protéine JEG80.
- **Figure 6 -**: Séquence nucléotidique du gène jeg80 (séquence codante comprise entre les nucléotides 1352 et 3526) et de l'ISjeg (Inverted repeat Sequence) (nucléotides 58 à 864). Les codons d'initiation et d'arrêt de la traduction sont soulignés. Le terminateur potentiel de transcription du gène jeg80 est indiqué par des flèches. Les séquences répétées en orientation inverse délimitant l'ISjeg sont encadrées.

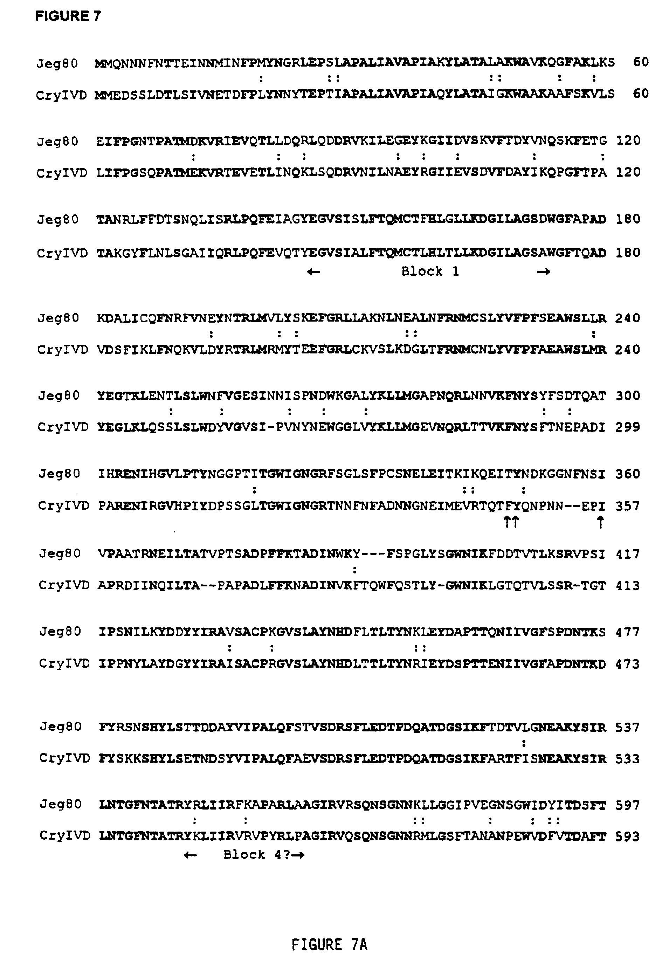

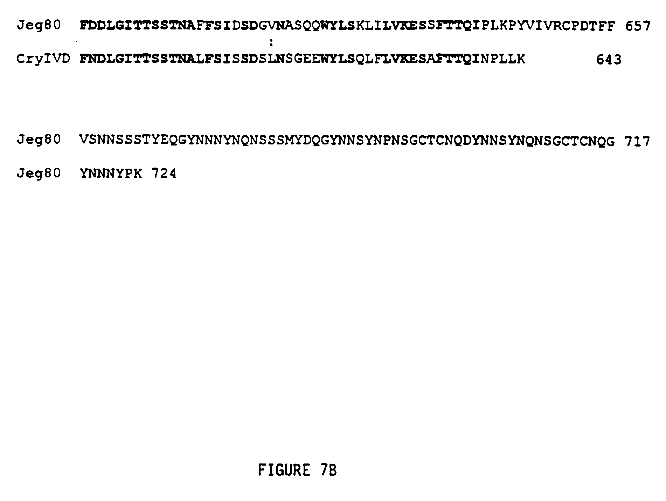

- **Figure 7 -**: comparaison des séquences de Jeg80 et de CryIVD. Les acides aminés identiques se correspondant sont encadrés. Les résidus identiques sur le plant fonctionnel sont indiqués par des points (les remplacements conservatifs sont I, L, V et M ; D et E ; Q et N ; K et R ; T et S ; G et A ; et F et Y). Les régions similaires aux blocs 1 et 4 présents dans toutes les protéines CryI, CryIII et certaines CryIV sont indiquées. Les flèches verticales représentent les sites de clivage pour les protéases dans la toxine CryIVD solubilisée (ref. 11). Les nombres indiquent le dernier résidu de chaque ligne pour chaque protéine.

### MATERIELS ET METHODES

### Souches bactériennes et plasmides

E.coli TG1 [K12, Δ(lac-proAB) supE thi hdsD F'(traD36 proA^{±} proB^{±} lacZΔ lacI^{g} lacZΔM15)] et pHT315 (Arantès, O. et al. (1991) Gene **108:**115-119) ont été utilisés comme hôtes de clonage et vecteurs respectivement. B thuringiensis ser. jegathesan 367 a été utilisée pour purifier les cristaux de type sauvage et l'ADN pour les expériences de clonage. B thuringiensis ser. thuringiensis SPL407 (Lereclus, D. et al. (1989) FEMS Microbiol. Lett. **60**:211-218) a été utilisée comme souche receptrice pour les expériences de transformation. La souche B thuringiensis ser. israelensis 4Q2-81(pHT640) a été utilisée comme source d'inclusions CryIVD (Poncet, S. et al. (1993) Appl. Environ. Microbiol. **59**:3928-3930).

B thuringiensis SPL407 a été transformée par électroporation selon la technique décrite dans la publication pré-citée (Lereclus, D. et al.) et E.coli a été transformée selon la description préalablement donnée dans la publication de Lederberg, E.M. et al. (1974) J. Bacteriol. **119**:1072-1074. Les concentrations en antibiotiques pour la sélection bactérienne étaient de 25 µg d'érythromycine par ml et 100 µg d'ampicilline par ml.

### Manipulations de l'ADN

Les enzymes de restriction, la ligase T4 de l'ADN et la phosphatase alcaline intestinale de veau ont été utilisées selon la description de Sambrook et al. (Sambrook, J. et al. (1989) a laboratory manual, 2 nd ed. Cold Spring Harbor Laboratory, Cold Spring Harbor, N.Y.) et selon les instructions des fabricants.

L'ADN total a été isolé de B thuringiensis ser. jegathesan selon la description de Delécluse, A. et al. (1991) J. Bacteriol. **173**:3374-3381. L'ADN du plasmide a été extrait à partir de E.coli par une procédure de lyse alcaline standard telle que décrite par Birnboim H.C. et al. (1979) Nucleic Acids Res. 7:1513-1523 et purifié de façon supplémentaire en utilisant le kit Qiagen (Qiagen GmbH, Allemagne). Des fragments d'ADN ont été purifiés sur gel d'agarose avec le kit de purification de l'ADN Prep A Gene (BioRad, Hercules, Californie).

Les expériences d'hybridation ont été conduites sur des filtres Hybond N⁺ (Amersham, Buckingamshire, Royaume-Uni). Les oligonucléotides étaient marqués avec de la fluoresceine en utilisant le système de marquage des oligonucléotides ECL 3' oligolabeling (Amersham).

Les séquences d'ADN ont été déterminées à partir des plasmides dénaturés en milieu alcalin en utilisant la méthode de terminaison de chaînes didésoxy (Sanger, F. et al. (1977) Proc. Natl. Acad. Sci. USA **74**:5463-5467) avec le kit Sequenase version 2.0 (U.S. Biochemical Corp., Cleveland, Ohio) et le α-³⁵S-dATP (>37 TBq/mmol ; Amersham). Une série d'oligonucléotides synthétiques (Eurogentec, Belgique) a été utilisée pour déterminer la séquence des deux brins.

Les logiciels d'analyse de séquences de Genetics Computer Group ont été utilisés (Université du Wisconsin, Madison).

### RESULTATS SCIENTIFIQUES

### 1 - Composition polypeptidique et activité des cristaux de Btjeg

La souche Btjeg 367 a été cultivée en milieu usuel glucosé à 30°C avec agitation pendant environ 72 heures jusqu'à la lyse des bactéries sporulantes. Les mélanges spores-cristaux ont été récoltés par centrifugation et les cristaux purifiés sur gradient de saccharose, selon la technique décrite par THOMAS et ELLAR (THOMAS and ELLAR, 1983, J. Cell. Sci., 60, 181-197). L'analyse de la composition polypeptidique de ces cristaux a été réalisée par électrophorèse sur gel de polyacrylamide-SDS suivie d'une coloration au bleu de Coomassie. Les résultats obtenus, représentés sur la figure 1 montrent que les cristaux de la souche Btjeg sont constitués de plusieurs polypeptides de 80, 72, 70, 66, 50, 37 et 28 kDa (dont certains pourraient être des produits de dégradation).

Des expériences d'immunodétection réalisées à l'aide d'un sérum dirigé contre les protéines de Bti ont permis de montrer que la protéine de 37 kDa est immunologiquement apparentée aux toxines de Bti ; une protéine d'environ 100 kDa, non détectée sur gel après coloration au bleu de Coomassie réagit également avec ce sérum.

Les cristaux de cette souche présentent, sur larves de Aedes aegypti, Anopheles stephensi, et Culex pipiens, une toxicité inférieure à celle obtenue avec les cristaux de Bti mais qui est néanmoins importante, comme indiqué dans le tableau suivant.

| Cristaux | CL50 en ng/ml à 24 h (intervalle de confiance) sur : | | |
|---|---|---|---|
| | Aedes aegypti | Anopheles stephensi | Culex pipiens |
| Bti 1884 | 20 (16-24) | 39 (29-49) | 20 (14-26) |
| Btjeg 367 | 240 (174-306) | 165 (119-211) | 77 (65-89) |

### 2 - Présence chez Btjeg des gènes codant pour des toxines ayant des propriétés similaires à celles de Bti

L'ADN total de la souche Btjeg 367 a été isolé selon la technique précédemment décrite (DELECLUSE et al., 1991, J. Bacteriol., 173, 3 374-3 381), puis hydrolysé par l'enzyme EcoRI. Les fragments obtenus ont été séparés par électrophorèse sur gel d'agarose 0,6 %, puis transférés sur une membrane de nylon (Hybond N+, Amersham).

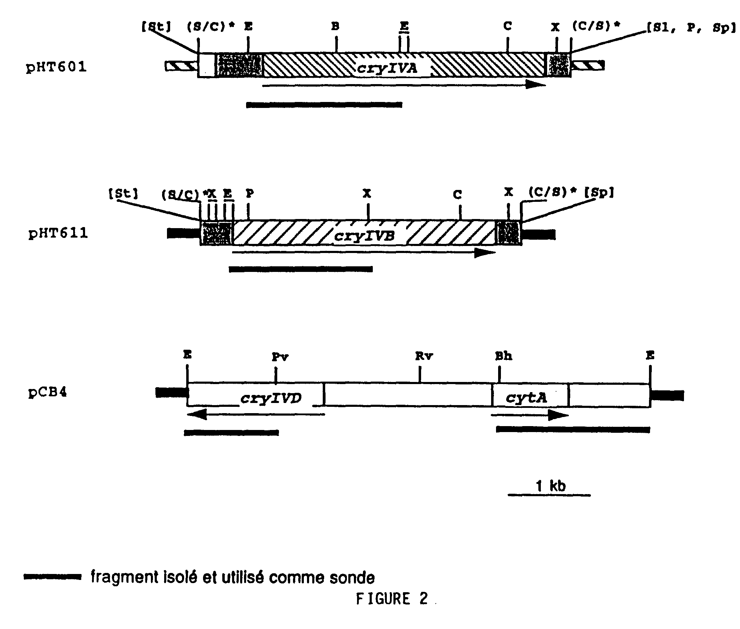

Par ailleurs, les gènes cryIVA, CryIVD et CytA de Bti ont été obtenus après hydrolyse des plasmides recombinants pHT606, pHT611, et pCB4, comme indiqué dans la figure 2. Les fragments d'ADN contenant les gènes de Bti ont été purifiés après séparation sur gel d'agarose puis marqués à la peroxydase à l'aide du kit "ELC direct nucleic acid" labelling (Amersham), et utilisés dans des expériences d'hybridation avec l'ADN total hydrolysé de la souche Btjeg.

Aucune hybridation n'a été obtenue entre l'ADN de la souche Btjeg et les gènes de Bti, tout au moins dans les conditions d'hybridation utilisées (80 % d'homologies).

### 3 - Clonage des gènes codant pour les toxines de la souche Btjeg

### a) Détermination des séquences acides-aminés des protéines de Btjeg

Les séquences amino-terminales des protéines de 80, 66 et 37 kDa ont été déterminées au laboratoire de microséquençage de l'Institut Pasteur en utilisant un séquenceur automatique (Applied Biosystems) après transfert de la protéine sur les membranes Problot (Applied Biosystems) ; l'enchaînement ainsi obtenu est donné dans le tableau ci-dessous.

Des oligonucléotides correspondant aux protéines JEG80, JEG66 et JEG37 (séquences soulignées) ont été synthétisés, en utilisant le code génétique déterminé pour plusieurs gènes de B. thuringiensis et en incorporant de l'inosine à chaque ambiguité. La séquence de ces oligonucléotides est représentée dans le tableau.

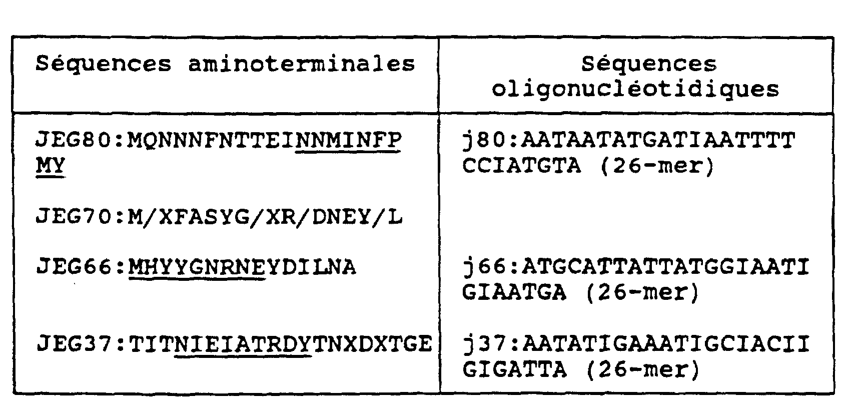

Dans ces séquences, "I" représente la deoxyinosine, utilisée comme base neutre pour toutes les positions pouvant correspondre à trois ou quatre nucléotides.

### b) Hybridation des oligonucléotides sur l'ADN total de la souche Btjeg

Des expériences d'hybridation ont été réalisées entre les oligonucléotides marqués à la fluorescéine et l'ADN total de la souche Btjeg, hydrolysé par EcoRI, HindIII, XbaI ou PstI. La technique de sondes froides (3' oligonucleotide labelling system, Amersham) a été utilisée. Les résultats obtenus sont indiqués dans le tableau ci-dessous.

| Oligonucléotides | Température d'hybridation | Taille des fragments | | | |
|---|---|---|---|---|---|
| | | EcoRI | HindIII | XbaI | PstI |
| j80 | 42°C | 2kb | 4kb | 12kb | 12kb |
| j66 | 47°C | 7kb | 14kb | 10kb | 14kb |

La sonde a hybride spécifiquement à 42°C à un fragment de restriction HindIII unique d'environ 4 kb et à un fragment de restriction EcoRI unique d'environ 2 kb.

### c) Clonage du gène codant pour la protéine JEG80

Une banque d'ADN de la souche Btjeg a été construite chez E. coli TGI en utilisant le plasmide bifonctionnel pHT315 (ARANTES and LERECLUS, 1991, Gene, 108, 115-119) comme vecteur de clonage. L'ADN total de la souche Btjeg a été hydrolysé par HindIII, puis soumis à une électrophorèse sur gel d'agarose. Des fragments d'environ 2 à 6 kb ont été purifiés et clonés dans ce vecteur. Les fragments HindIII de 3 à 5 kb ont été insérés dans le site HindIII du vecteur navette pHT315 traité avec la phosphatase alcaline. Les clones recombinants obtenus après transformation de la souche de E. coli TGI avec les mélanges de ligation obtenus ont été testés pour leur hybridation avec l'oligonucléotide j80 marqué. Sur 1 000 clones recombinants obtenus, 5 présentaient une réaction positive. Un clone recombinant, JEG80.1, a été sélectionné et analysé. Ce clone contient un plasmide recombinant (pJEG80.1) de 10,9 kb ; le fragment HindIII d'ADN inséré a une taille de 4,3 kb.

### 4 - Analyse du clone recombinant JEG80.1

### a) Détermination de la carte de restriction du plasmide pJEG80.1

La carte de restriction du plasmide pJEG80.1 a été déterminée et est représentée sur la figure 3. Des expériences d'hybridation réalisées avec l'oligonucléotide j80 ont permis de localiser la position de cet oligonucléotide sur le fragment HindIII de 4,3 kb. Par ailleurs, des expériences de PCR, réalisées avec les combinaisons d'oligonucléotides j80 + universel et j80 + réverse ont permis de localiser plus précisément le début du gène codant pour la protéine JEG80 (jeg80) ainsi que son sens de transcription (figure 3).

### b) Détermination de la séquence du gène jeg80

La séquence nucléotidique du gène jeg80 est réalisée par la technique de SANGER sur le plasmide pJEG80.1 préalablement dénaturé à la soude. Les amorces utilisées sont l'oligonucléotide j80, l'oligonucléotide réverse ou des oligonucléotides déduits des séquences lues. Une séquence partielle (938 pb à partir de l'extrémité 5' du gène), est représentée sur la figure 4 avec la séquence d'acides aminés correspondante.

La séquence pJEG80.1 dans la région contenant le gène pour la protéine de 80 kDa (désignée jeg80) a été déterminée sur les deux brins (figure 5). On a trouvé un cadre ouvert de lecture codant pour un polypeptide de 724 résidus avec une masse moléculaire calculée de 81.293 Da. La séquence a été examinée pour déterminer toute région qui pourrait s'apparenter à des structures de promoteur de B. thuringiensis. Aucune séquence de promoteur n'a été trouvée dans la séquence comportant environ 50 nucléotides en amont du codon start. En aval du codon stop (position 2249 à 2282, Figure 5), des séquences inversées et répétées ont été identifiées, séquences capables de former une structure en boucle avec un ΔG = -76,9 kJ/mol calculé selon des règles définies par Tinoco et al. (Tinoco, I.J. et al. (1973) Nature (Londres) New Biol. **246**:40-41.

Cette structure peut agir en tant que terminateur de transcription. Douze nucléotides en amont du codon de départ, constituant une séquence AAAGAAGAGGG a été identifiée comme constituant un site de liaison au ribosome (figure 5).

La séquence en acides aminés déduite de la séquence nucléotidique obtenue a été comparée aux séquences d'autres protéines présentes dans la banque de données Swiss Prot. Cette analyse a permis de montrer que la protéine Jeg80 présente une similitude de l'ordre de 67% avec la partie N-terminale de la protéine CryIVD de Bti ; sur la totalité de la séquence d'acides aminés, la similitude est d'environ 58% (figure 7) et à un degré moindre (36 %) avec les protéines CryII de Bt kurstaki.

Cinq blocs sont conservés parmi les toxines CryI, CryIII et la plupart des toxines CryIV (Höfte, H. et al. (1989) Microbiol. Rev. 53:242-255. Cependant seul le bloc 1 a été trouvé dans Jeg80 (figure 7), avec une région arginine alternée reminiscente du bloc 4 et cependant similaire (figure 7). Jeg80 présente une chaîne de 82 acides aminés comprenant cinq résidus cystéine à sa partie COOH-terminale, absente de CryIVD. La région sensible à l'activité de la protéase de CryIVD (Dai, S.-M. et al. (1993) Insect. Biochem. Molec. Biol. 23:273-283 localisée dans le milieu de la protéine (acides aminés 348-357, figure 7) n'est pas conservée dans Jeg80.

### c) Expression du gène jeg80 dans la souche de Bt 407 cry- (également désignée par SPL 407).

Le plasmide pJEG80.1 a été introduit dans la souche de Bt 407 cry- par électroporation selon la technique décrite par LERECLUS et al. (LERECLUS et al. 1989, FEMS Microbiol. Lett. 60, 211-218). Un transformant, 407 (pJEG80.1), a été sélectionné pour analyse complémentaire. Ce clone produit pendant la sporulation des inclusions visibles en microscopie optique. Aucune inclusion de ce type n'était présente dans des cellules contenant le vecteur pHT315 seul. Le produit d'expression de jeg80 a été analysé par SDS PAGE puis coloration avec du bleu de Coomassie brillant (figure 1). Le polypeptide principal des inclusions, purifié à partir de la souche recombinante 407 (pJEG80.1) avait un poids moléculaire d'environ 80 kDa (figure 1, ligne 3), le même que celui du plus gros polypeptide des cristaux de la souche sauvage 367 (figure 1, ligne 2). Ces inclusions ont été purifiées sur gradient de saccharose : elles sont constituées uniquement de la protéine Jeg80 (figure 1). Des réactions immunologiques ont été testées entre le polypeptide Jeg80 et les protéines du cristal de B. thuringiensis ser jegathesan, B. thuringiensis ser israelensis, B. thuringiensis ser medellin et darmstadiensis. Les résultats de la réaction immunologique sont rapportés à la figure 1B. Cette protéine réagit avec des anticorps dirigés contre les cristaux totaux de Btjeg ainsi qu'avec le sérum anti-cristaux de Bti, et anti-cristaux totaux de la souche Bt medellin (autre souche active sur moustiques, d'un nouveau sérotype, H30, récemment identifiée).

### d) Activité larvicide de la protéine JEG80

Les cristaux purifiés de la souche 407 (pJEG80.1) ont été testés pour leur activité sur larves de moustiques, en comparaison avec les cristaux provenant de la souche Btjeg contenant toutes les protéines. Des tests ont aussi été faits avec la souche 4Q2-81 (pHT 640) qui produit seulement la protéine CryIVD. Des résultats préliminaires indiquent que la protéine Jeg80 est active sur les trois espèces de moustiques testées : A. aegypti, A. stephensi et C. pipiens. La toxicité de la protéine Jeg80 est plus élevée avec C. pipiens (tableau 1). Par ailleurs, le niveau de toxicité obtenu pour la protéine Jeg80 seule est comparable sur A. stephensi à celui observé avec les cristaux de Btjeg.

La protéine Jeg80 est plus toxique que la souche sauvage vis-à-vis de A. aegypti et aussi toxique vis-à-vis de C. pipiens et A. stephensi. De plus et malgré des similitudes avec CryIVD, la protéine Jeg80 est plus toxique que CryIVD : environ 10 fois plus vis-à-vis de A. aegypti et A. stephensi et 40 fois plus vis-à-vis de C. pipiens.

L'activité toxique a été testée dans les conditions suivantes : les moustiques utilisés ont été maintenus en laboratoire à 26°C, dans une atmosphère humide à 80% et pendant une période jour-nuit de 14h-10h. Les larves ont été soumises à un traitement avec de l'eau déchlorurée et nourries avec des biscuits pour chat du commerce. Les inclusions purifiées ont été diluées dans des coupelles en plastique contenant 150 ml d'eau déionisée et testées en double contre 25 larves A. aegypti et C. pipiens de stade quatre et A. stephensi de stade trois. Chaque test a été répété au moins cinq fois. La mortalité des larves a été enregistrée après 48 heures et les doses létales (LC_{50S}) ont été déterminées par analyse Probit.

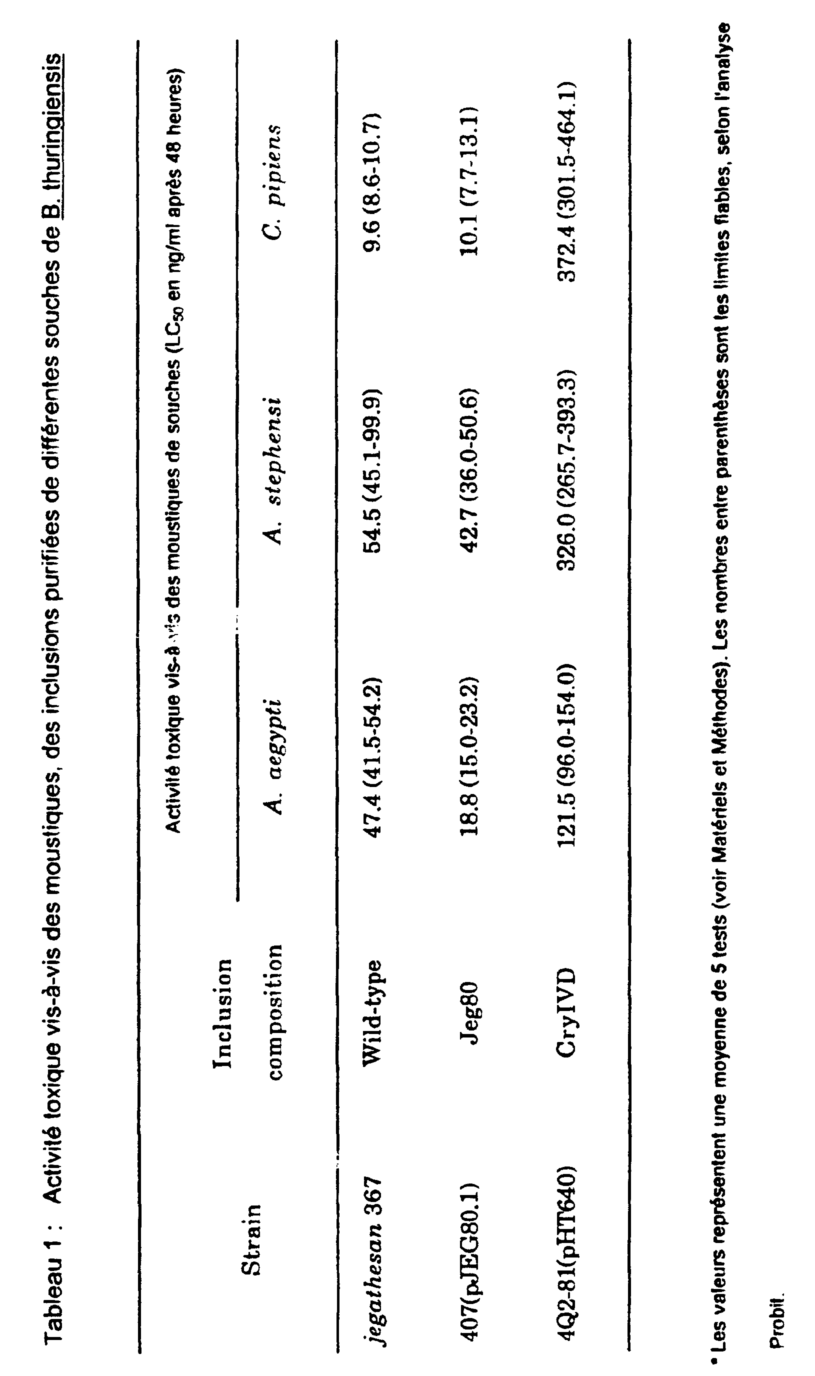

On a décrit précédemment le clonage et la caractérisation d'un nouveau gène de B. thuringiensis, le gène jeg80 de la souche jegathesan 367. Le gène jeg80 code pour une protéine de poids moléculaire 81.293 Da.

La protéine Jeg80 est similaire à la toxine larvicide vis-à-vis des moustiques CryIVD de B. thuringiensis ser. israelensis. Il s'agit même de la seule protéine connue présentant des similitudes avec CryIVD, suggérant que ces deux protéines ont évolué à partir d'un ancêtre commun. La protéine Jeg80 partage également une faible similitude avec les protéines CryII, comparable à la ressemblance existant entre CryIV et CryII. Cependant, il existe des différences essentielles entre Jeg80 et CryIVD, en particulier au niveau de la partie carboxy-terminale de la protéine. Jeg80 comporte une séquence de 82 acides aminés incluant 5 résidus cystéine, séquence qui est absente de CryIVD. Bietlot et al. (Bietlot, H.P. et al. (1990) Biochem. J. **267**:309-315) ont décrit l'importance de tels résidus dont la stabilité de plusieurs δ-endotoxines, produites par différentes souches de B. thuringiensis. Cette structure pourrait être essentielle pour la formation du cristal et l'activité insecticide. La mutagénèse pourrait être utilisée pour identifier le rôle de cette partie carboxy-terminale originale de Jeg80. Il existe également des différences importantes entre les régions flanquantes des gènes cryIVD et jeg80. Le gène cryIVD est le deuxième gène d'un opéron contenant deux autres gènes, p19 et p20 (Adams, L.F. et al. (1989) J. Bacteriol. **171**:521-530 et Dervyn, E. et al. (1995) J. Bacteriol. **177**:2283-2291). Bien que les peptides correspondants, P19 et P20 ne soient pas essentiels pour l'expression de CryIVD, ils pourraient agir comme protéine chaperon pour stabiliser certains composants du cristal de B. thuringiensis ser. israeliensis (Chang, C. et al. (1993) Appl. Environ. Microbiol. **59**:815-821 ; Dervyn, E. et al. (1995) J. Bacteriol. **177**:2283-2291 et Wu, D. et al. (1994) Mol. Microbiol. **13**:965-972). Aucun environnement de ce type n'a été reconnu pour le gene jeg80 : aucun homologue à p20 n'a été trouvé en aval de jeg80. Cependant le fragment d'ADN clone dans le plasmide Jeg80.1 pourrait être trop petit pour contenir le point de départ d'un autre gène. De façon similaire aucun gène apparenté à p19 n'a été trouvé dans le fragment de 1 kb en amont de Jeg80. Au contraire à une distance de 550 pb en amont du gène jeg80, un cadre ouvert de lecture orienté dans la direction opposée a été identifié. Des comparaisons de la séquence d'acides aminés déduite avec d'autres à l'aide de la banque de données Swiss Prot a révélé des similitudes avec la transposase de la séquence d'insertion IS240 de B. thuringiensis ser. israeliensis (Delécluse, A. (1989) Plasmid **21**:71-78). Deux copies de IS240 flanquent le gène cryIVA dans la variété israeliensis (Bourgouin, C. et al. (1988) J. Bacteriol. **170**:3575-3583) mais aucune n'a été trouvée au voisinage du gène cryIVD bien qu'un variant IS231 ait été trouvé en aval du gène p20 (Adams, L.F. et al. (1989) J. Bacteriol. **171**:521-530). Des éléments d'insertion peuvent rendre compte de la dispersion des gènes des toxines entre les différences souches de B. thuringiensis. Le gène cryIVD est transcrit à partir de deux promoteurs, reconnus par l'ARN polymérase associé avec des facteurs σ35 ou σ28 de B. thuringiensis (Dervyn, E. et al. (1995) J. Bacteriol. 177:2283-2291). L'analyse de la séquence de la région en amont du gène jeg80 n'a pas révélé de promoteur consensus de B. thuringiensis. Il est possible que jeg80 soit transcrit à partir d'un promoteur reconnu par les facteurs σ, différents de σ35 et σ28 ou que le promoteur soit localisé très en amont du gène jeg80, c'est-à-dire en amont de la séquence apparentée à la séquence IS240. La protéine Jeg80 réagit de façon croisée avec des anticorps dirigés contre CryIVD et CryIVA. Bien que les gènes jeg80 et cryIVA ne présentent pas de similitudes importantes, les protéines peuvent néanmoins partager des domaines similaires. La protéine Jeg80 a également réagi avec un sérum contre les protéines totales de B. thuringiensis ser. medellin. Des expériences préalables n'avaient pas révélé de polypeptides analogues du polypeptide CryIVD dans cette souche (Orduz, D. et al. (1994) Microbiol. Biotechnol. **40**:794-799 et Ragni, A. et al. (Submitted for publication).

Les inclusions composées de la protéine Jeg80 seule étaient aussi toxiques que les inclusions de la souche sauvage B. thuringiensis ser. jegathesan contre les larves des souches C. pipiens et A. stephensis et plus toxiques que la souche sauvage sur les larves de A. aegypti. Ceci constitue le premier résultat de l'existence d'une protéine ayant une activité toxique vis-à-vis des larves de moustique, capable sous forme isolée de présenter une activité similaire à celle d'un mélange de différents polypeptides. Dans le cas de B. thuringiensis ser. israeliensis, des polypeptides isolés et même des combinaisons de deux ou trois composants du cristal sont moins toxiques que les cristaux de type sauvage (Angsuthanasombat, C. et al. (1987) Mol. Gen. Genet. **208**:384-389 ; Delécluse, A. et al. (1993) Appl. Environ. Microbiol. **177**:2283-2291 ; Poncet, S. et al. (J. Invertebr. Pathol.: in press) et Wu, D. et al. (1994) Mol. Microbiol. **13**:965-972). La forte activité de la souche israeliensis est due à des interactions synergiques entre différents polypeptides du cristal. Pour la souche jegathesan de telles interactions ne sont pas exclues bien que la protéine Jeg80 apparaisse comme un contributeur prédominant à la toxicité. Cependant Jeg80 n'est pas le composant principal des cristaux de jegathesan. D'autres polypeptides dans les cristaux, probablement des protéines de 65 kDa ou 37 kDa ou les deux sont responsables de l'activité complémentaire.

Jeg80 est beaucoup plus toxique (6 à 40 fois plus toxique selon les espèces de moustiques testés) que CryIVD, malgré leur forte similitude. Cette différence d'activité pourrait refléter des modes d'action différents des deux toxines. Cette différence pourrait être exploitée dans le développement d'insecticides.

## Revendications

1. Polynucléotide **caractérisé en ce qu'**il correspond au fragment HindIII d'environ 4,3 kb pouvant être obtenu, à partir du plasmide pJEG80.1 déposé à la CNCM le 23 août 1994 sous le numéro I-1469 ou **en ce qu'**il est capable d'hybrider dans des conditions stringentes avec le fragment HindIII de ce plasmide.

2. Polynucléotide selon la revendication 1, **caractérisé en ce qu'**il est compris dans le fragment HindIII d'environ 4,3 kb et **en ce qu'**il code pour un polypeptide ayant une activité toxique vis-à-vis d'insectes de la famille des Diptères.

3. Polynucléotide selon la revendication 1 ou la revendication 2, **caractérisé en ce qu'**il comprend l'enchaînement de nucléotides suivant ou l'enchaînement contenu dans le suivant, compris entre les nucléotides 325 et 1260 :

4. Polynucléotide, **caractérisé en ce qu'**il répond à la séquence du gène jeg80 représentée à la figure 5A entre les nucléotides 64 et 2238.

5. Polynucléotide, **caractérisée en ce qu'**il hybride dans des conditions stringentes avec un polynucléotide selon l'une quelconque des revendications 1 à 4 et **en ce qu'**il code pour un polypeptide ayant une activité toxique vis-à-vis d'insectes de la famille des Diptères.

6. Polynucléotide selon l'une quelconque des revendications 1 à 4, **caractérisé en ce qu'**il code pour un polypeptide ayant une activité toxique vis-à-vis d'insectes de la famille des Diptères et ayant un poids moléculaire d'environ 80 kDa.

7. Polynucléotide de *B. thuringiensis jegathesan*, **caractérisé en ce qu'**il hybride dans des conditions stringentes, à une température de 42°C et avec un deuxième lavage après hybridation dans du 1xSSC - 0,1% SDS, avec l'oligonucléotide j66 ayant la séquence suivante : et **en ce qu'**il code pour un polypeptide d'environ 66 kDa comprenant la séquence MHYYGNRNEYDILNA qui, lorsqu'il s'associe avec un polypeptide codé par un polynucléotide selon l'une quelconque des revendications 1 à 6, participe avec ce dernier à l'activité toxique vis à vis des insectes de la famille des Diptères.

8. Polynucléotide de *B. thuringiensis jegathesan*, **caractérisé en ce qu'**il hybride dans des conditions stringentes, à une température de 42°C et avec un deuxième lavage après hybridation dans du 1xSSC - 0,1% SDS, avec l'oligonucléatide j37 ayant la séquence suivante : et **en ce qu'**il code pour un polypeptide d'environ 37 kDa comprenant la séquence NIEIATRDY qui, lorsqu'il s'associe avec un polypeptide codé par un polynucléotide selon l'une quelconque des revendications 1 à 6, participe avec ce dernier à l'activité toxique vis à vis des insectes de la famille des Diptères.

9. Vecteur de clonage ou d'expression, **caractérisé en ce qu'**il comprend un polynucléotide selon l'une quelconque des revendications 1 à 8.

10. Vecteur selon la revendication 9. **caractérisé en ce qu'**il s'agit du plasmide pJEG80.1 déposé à la CNCM le 23 août 1994 sous te numéro 1-1469.

11. Polypeptide **caractérisé en ce qu'**il s'agit du produit d'expression d'un polynucléotide selon l'une quelconque des revendications 1 à 8.

12. Polypeptide selon la revendication 11, **caractérisé en ce qu'**il est codé par le gène jeg80 représenté à la figure 5A.

13. Polypeptide ayant une activité toxique vis-à-vis d'insectes de la famille des diptères, **caractérisé en ce qu'**il répond à la séquence d'acides aminés présentée à la figure 5B.

14. Polypeptide selon la revendication 12 ou la revendication 13, **caractérisé en ce qu'**il comprend la séquence d'acides aminés suivante, **en ce qu'**il a un poids moléculaire d'environ 80 kDa et **en ce qu'**il a une activité toxique vis-à-vis d'insectes de la famille des diptères.

15. Composition ayant une activité toxique vis-à-vis d'insectes de la famille des diptères, **caractérisée en ce qu'**elle comprend à titre de principe actif, au moins un polypeptide selon l'une quelconque des revendications 11 à 14.

16. Cellule recombinante procaryote ou eucaryote, **caractérisée en ce qu'**elle contient un vecteur de clonage ou d'expression selon la revendication 9 ou la revendication 10.

17. Utilisation comme sonde ou amorce d'un polynucléotide, hybridant dans des conditions stringentes avec un polynucléotide selon l'une quelconque des revendications 1 à 6, et choisi dans le groupe constitué :
- du fragment HindIII d'environ 4,3 kb pouvant être obtenu à partir du plasmide pJEG80.1 déposé à la CNCM le 23 août 1994 sous le numéro 1-1469,
- du fragment HindIII-NdeI d'environ 2,2 kb pouvant être obtenu à partir du plasmide pJEG80.1,
- du fragment compris entre les nucléotides 1 et 124 de l'enchaînement représenté à la figure 4, et
- du fragment jeg 80 répondant à l'enchaînement AATAATATGATIAATTTTCCIATGTA

## Patentansprüche

1. Polynucleotid, **dadurch gekennzeichnet, dass** es dem HindIII-Fragment von etwa 4,3 kb entspricht, das ausgehend von dem Plasmid pJEG80.1 erhalten werden kann, das am 23. August 1994 unter der Nummer I-1469 bei der CNCM hinterlegt wurde, oder **dadurch**, dass es in der Lage ist, unter stringenten Bedingungen mit dem HindIII-Fragment dieses Plasmids zu hybridisieren.

2. Polynucleotid gemäß Anspruch 1, **dadurch gekennzeichnet, dass** es in dem HindIII-Fragment von etwa 4,3 kb enthalten ist und **dadurch**, dass es ein Polypeptid codiert, das eine toxische Wirkung gegenüber Insekten der Familie der Dipteren aufweist.

3. Polynucleotid gemäß Anspruch 1 oder Anspruch 2, **dadurch gekennzeichnet, dass** es die folgende Abfolge von Nucleotiden enthält oder die Abfolge umfasst, die in dem Folgenden enthalten ist, enthalten zwischen den Nucleotiden 325 und 1260:

4. Polynucleotid, **dadurch gekennzeichnet, dass** es der Sequenz des Gens jeg80, zwischen den Nucleotiden 64 und 2238, dargestellt in Figur 5A, entspricht.

5. Polynucleotid, **dadurch gekennzeichnet, dass** es unter stringenten Bedingungen mit einem Polynucleotid gemäß einem der Ansprüche 1 bis 4 hybridisiert, und **dadurch**, dass es ein Polypeptid codiert, das eine toxische Wirkung gegenüber Insekten der Familie der Dipteren aufweist.

6. Polynucleotid, gemäß einem der Ansprüche 1 bis 4, **dadurch gekennzeichnet, dass** es ein Polypeptid codiert, das eine toxische Wirkung gegenüber Insekten der Familie der Dipteren aufweist, und **dadurch**, dass es ein Molekulargewicht von etwa 80 kDa aufweist.

7. Polynucleotid aus *B. thuringiensis jegathesan*, **dadurch gekennzeichnet, dass** es unter stringenten Bedingungen bei einer Temperatur von 42°C und bei einem zweiten Waschen nach der Hybridisierung in 1 x SSC - 0,1% SDS mit dem Oligonucleotid j66 mit der folgenden Sequenz: hybridisiert, und **dadurch**, dass es ein Polypeptid mit etwa 66 kDa codiert, umfassend die Sequenz MHYYGNRNEYDILNA, das, wenn es eine Verbindung mit einem Polypeptid eingeht, das von einem Polynucleotid gemäß einem der Ansprüche 1 bis 6 codiert wird, mit dem Letzteren an der toxischen Wirkung gegenüber Insekten der Familie der Dipteren teilhat.

8. Polynucleotid aus *B. thuringiensis jegathesan*, **dadurch gekennzeichnet, dass** es unter stringenten Bedingungen bei einer Temperatur von 42°C und bei einem zweiten Waschen nach der Hybridisierung in 1 x SSC - 0,1% SDS mit dem Oligonucleotid j37 mit der folgenden Sequenz: hybridisiert, und **dadurch**, dass es ein Polypeptid von etwa 37 kDa codiert, umfassend die Sequenz NIEIATRDY, das, wenn es mit einem Polypeptid eine Verbindung eingeht, das von einem Polynucleotid gemäß einem der Ansprüche 1 bis 6 codiert wird, mit dem Letzteren an der toxischen Wirkung gegenüber den Insekten der Familie der Dipteren teilhat.

9. Clonierungs- oder Expressionsvektor, **dadurch gekennzeichnet, dass** er ein Polynudeotid gemäß einem der Ansprüche 1 bis 8 umfasst

10. Vektor gemäß Anspruch 9, **dadurch gekennzeichnet, dass** es sich um das Plasmid pJEG80.1 handelt, das am 23. August 1994 unter der Nummer 1-1469 bei der CNCM hinterlegt wurde.

11. Polypeptid, **dadurch gekennzeichnet, dass** es sich um das Expressionsprodukt eines Polynucleotids gemäß einem der Ansprüche 1 bis 8 handelt.

12. Polypeptid gemäß Anspruch 11, **dadurch gekennzeichnet, dass** es vom Gen jeg80 codiert wird, das in Figur 5A dargestellt ist

13. Polypeptid, das eine toxische Wirkung gegenüber Insekten der Familie der Dipteren aufweist, **dadurch gekennzeichnet, dass** es der in Figur 5B dargestellten Aminosäuresequenz entspricht

14. Polypeptid gemäß Anspruch 12 oder Anspruch 13, **dadurch gekennzeichnet, dass** es die folgende Aminosäuresequenz umfasst, **dadurch**, dass es ein Molekulargewicht von etwa 80 kDa aufweist und **dadurch**, dass es eine toxische Wirkung auf Insekten der Familie der Dipteren aufweist:

15. Zusammensetzung, die eine toxische Wirkung auf Insekten der Familie der Dipteren aufweist, **dadurch gekennzeichnet, dass** sie als Wirkstoff mindestens ein Polypeptid gemäß einem der Ansprüche 11 bis 14 umfasst.

16. Rekombinante prokaryontische oder eukaryontische Zelle, **dadurch gekennzeichnet, dass** sie einen Clonierungs- oder Expressionsvektor gemäß Anspruch 9 oder Anspruch 10 enthält.

17. Verwendung eines Polynucleotids als Sonde oder Primer, das unter stringenten Bedingungen mit einem Polynucleotid gemäß einem der Ansprüche 1 bis 6 hybridisiert und ausgewählt ist aus der Gruppe bestehend aus:
- dem HindIII-Fragment von etwa 4,3 kb, das ausgehend von dem Plasmid pJEG80.1 erhalten werden kann, das am 23. August 1994 unter der Nummer I-1469 bei der CNCM hinterlegt wurde,
- dem HindIII-Ndel-Fragment von etwa 2,2 kb, das ausgehend vom Plasmid pJEG80.1 erhalten werden kann,
- dem Fragment, das zwischen den Nudeotiden 1 und 124 der in Figur 4 dargestellten Abfolge enthalten ist und
- dem Fragment jeg80, das der Abfolge AATAATATGATIAATTTTCCIATGTA entspricht.

## Claims

1. Polynucleotide **characterized in that** it corresponds to the HindIII fragment of about 4.3 kb which can be obtained from the plasmid pJEG80.1 deposited with the CNCM on August 23, 1994 under the number I-1469 or is capable of hybridizing with this plasmid under stringent conditions.

2. Polynucleotide according to claim 1, **characterized in that** it is comprised in the HindIII fragment of about 4.3 kb and **in that** it encodes a polypeptide having a toxic activity against insects of the Diptera family.

3. Polynucleotide according to claim 1 or claim 2, **characterized in that** it comprises the following nucleotide concatenation or the concatenation contained in the following included between the nucleotides 325 and 1260:

4. Polynucleotide, **characterized in that** it corresponds to the sequence of the jeg80 gene shown in Figure 5A between the nudeotides 64 and 2238.

5. Polynucleotide, **characterized in that** it hybridizes under stringent conditions with a polynucleotide according to any one of claims 1 to 4 and **in that** it encodes a polypeptide having a toxic activity against insects of the Diptera family.

6. Polynucleotide according to any one of claims 1 to 4, **characterized in that** it encodes a polypeptide having a toxic activity against insects of the Diptera family and having a molecular weight of about 80 kDa.

7. *B. thuringiensis jegathesan* polynucleotide **characterized in that** it hybridizes under stringent conditions, at a temperature of 42° C, and with a second washing after hybridization being made in SSC 1X-SDS0.1%, with the oligonucleotide j66 which has the following sequence: and **in that** it codes for a polypeptide of about 66 kDa comprising the MHYYGNRNEYDILNA sequence which, in combination with a polypeptide encoded by a polynucleotide according to any one of the claims 1 to 6, contributes with this latter to the toxic activity against insects of the Diptera family.

8. *B. thuringiensis jegathesan* polynucleotide **characterized in that** it hybridizes under stringent conditions, at a temperature of 42° C, and with a second washing after hybridization being made in SSC 1X-SDS0.1%, with the oligonucleotide j37 which has the following sequence: and **in that** it codes for a polypeptide of about 37 kDa comprising the NIEIATRDY sequence which, in combination with a polypeptide encoded by a polynucleotide according to any one of the claims 1 to 6, contributes with this latter to the toxic activity against insects of the Diptera family.

9. Cloning or expression vector, **characterized in that** it comprises a polynucleotide according to any one of claims 1 to 8.

10. Vector according to claim 9, **characterized in that** it is the plasmid pJEG80.1 deposited with the CNCM on 23 August 1994 under the number I-1469.

11. Polypeptide **characterized in that** it is the expression product of a polynucleotide according to any one of claims 1 to 8.

12. Polypeptide according to claim 11, **characterized in that** it is encoded by the gene jeg80 shown in Figure 5A.

13. Polypeptide having a toxic activity against insects of the Diptera family, **characterized in that** it corresponds to the amino acid sequence shown in Figure 5B.

14. Polypeptide according to claim 12 or claim 13, **characterized in that** it has the following amino acid sequence, **in that** it has a molecular weight of about 80 kDa and that it has a toxic activity against insects of the Diptera family.

15. Composition having a toxic activity against insects of the Diptera family, **characterized in that** it contains as active ingredient at least one polypeptide according to any one of claims 11 to 14.

16. Prokaryotic or eukaryotic recombinant cell, **characterized in that** it contains a cloning or expression vector according to claim 9 or claim 10.

17. Use, as a probe or a primer, of a polynucleotide hybridizing under stringent conditions with a polynucleotide according to any one of claims 1 to 6, and selected from the group consisting of:
- the HindIII fragment of about 4.3 kb which can be obtained from the plasmid pJEG80.1 deposited with the CNCM on 23 August 1994 under the number I-1469;
- the HindIII-NdeI fragment of about 2.2 kb which can be obtained from the plasmid pJEG80.1;
- the fragment comprised between nucleotides 1 and 124 of the concatenation shown in Figure 4;
- the fragment jeg80 corresponding to the concatenation AATAATATGATTAATTTTCCTATGTA.


### LISTE DE SEQUENCES

<110> INSTITUT PASTEUR
<120> NOUVEAUX POLYPEPTIDES A ACTIVITE TOXIQUE VIS-A-VIS D'INSECTES DE LA FAMILLE DES DIPTERES
<130> B2571AA - I. PASTEUR
<140> 95928541.2-
   <141> 1995-08-24
<150> FR 9410299
   <151> 1994-08-25
<150> PCT/FR9501116
   <151> 1995-08-24
<160> 13
<170> PatentIn Ver. 2.1
<210> 1
   <211> 26
   <212> ADN
   <213> Séquence artificielle
<220>
   <223> Description de la séquence artificielle: oligonucléotide j80
<220>
   <221> misc_feature
   <222> (1)..(26)
   <223> n = inosine (i)
<400> 1
   aataatatga tnaattttcc natgta 26
<210> 2
   <211> 26
   <212> ADN
   <213> Séquence artificielle
<220>
   <223> Description de la séquence artificielle: oligonucléotide j66
<220>
   <221> misc_feature
   <222> (1)..(26)
   <223> n = inosine (i)
<400> 2
   atgcattatt atggnaatng naatga 26
<210> 3
   <211> 26
   <212> ADN
   <213> Séquence artificielle
<220>
   <223> Description de la séquence artificielle: oligonucléotide j37
<220>
   <221> misc_feature
   <222> (1)..(26)
   <223> n = inosine (i)
<400> 3
   aatatngaaa tngcnacaag agatta 26
<210> 4
   <211> 20
   <212> PRT
   <213> Séquence artificielle
<220>
   <223> Description de la séquence artificielle: séquence aminoterminale de JEG80
<400> 4
   Met Gln Asn Asn Asn Phe Asn Thr Thr Glu Ile Asn Asn Met Ile Asn
   1 5 10 15
   Phe Pro Met Tyr
   20
<210> 5
   <211> 15
   <212> PRT
   <213> Séquence artificielle
<220>
   <223> Description de la séquence artificielle: séquence aminoterminale de JEG66
<400> 5
   Met His Tyr Tyr Gly Asn Arg Asn Glu Tyr Asp Ile Leu Asn Ala
   1 5 10 15
<210> 6
   <211> 20
   <212> PRT
   <213> Séquence artificielle
<220>
   <223> Description de la séquence artificielle: séquence aminoterminale de JEG37
<400> 6
   Thr Ile Thr Asn Ile Glu Ile Ala Thr Arg Asp Tyr Thr Asn Xaa Asp
   1 5 10 15
   Xaa Thr Gly Glu
   20
<210> 7
   <211> 26
   <212> ADN
   <213> Séquence artificielle
<220>
   <223> Description de la séquence artificielle: oligonucléotide j37
<220>
   <221> misc_feature
   <222> (1)..(26)
   <223> n = inosine (i)
<400> 7
   aatatngaaa tngcnacnng ngatta                                    26
<210> 8
   <211> 1254
   <212> ADN
   <213> Bacillus thuringiensis
<220>
   <221> CDS
   <222> (325)..(1254)
<400> 8
   gatgtgagga ttattacgtg aatactgatt atgataattt tgaaaagaaa tcatatttgc 60
   aaccagaatc atcatatgat tacatgtcag aagaaaatca caaagttcca ttaattagat 120
   gtgaattcct atttttattt cagtccagaa tcctgaataa tggaaattaa atgcacccta 180
   tgatttataa atatatgtac ctttaaaaca agaataatta taactgtata aaaataggta 240
   tactattgga aaacaaaaaa gttaattatg aaaagatttc gtttatatta gtaaattgtt 300
   taaagaagag ggggcatgtt ttaa atg caa aat aac aac ttt aat acc aca    351
                              Met Gln Asn Asn Asn Phe Asn Thr Thr
                                1               5
   gaa att aat aat atg att aat ttc cct atg tat aat ggt aga tta gaa   399
   Glu Ile Asn Asn Met Ile Asn Phe Pro Met Tyr Asn Gly Arg Leu Glu
    10                  15                  20                  25
   cct tct cta gct cca gca tta ata gca gta gct cca att gct aaa tat   447
   Pro Ser Leu Ala Pro Ala Leu Ile Ala Val Ala Pro Ile Ala Lys Tyr
                   30                  35                  40
   tta gca aca gct ctt gct aaa tgg gct gta aaa caa ggg ttt gca aaa   495
   Leu Ala Thr Ala Leu Ala Lys Trp Ala Val Lys Gln Gly Phe Ala Lys
               45                  50                  55
   tta aaa tcc gag ata ttc ccc ggt aat acg cct gct act atg gat aag   543
   Leu Lys Ser Glu Ile Phe Pro Gly Asn Thr Pro Ala Thr Met Asp Lys
           60                  65                  70
   gtt cgt att gag gta caa aca ctt tta gac caa aga tta caa gat gac   591
   Val Arg Ile Glu Val Gln Thr Leu Leu Asp Gln Arg Leu Gln Asp Asp
       75                  80                  85
   aga gtt aag att tta gaa ggt gaa tac aaa gga att att gac gtg agt   639
   Arg Val Lys Ile Leu Glu Gly Glu Tyr Lys Gly Ile Ile Asp Val Ser
    90                  95                 100                 105
   aaa gtt ttt act gat tat gtt aat caa tct aaa ttt gag act gga aca    687
   Lys Val Phe Thr Asp Tyr Val Asn Gln Ser Lys Phe Glu Thr Gly Thr
                   110                 115                 120
   gct aat agg ctt ttt ttt gat aca agt aac caa agc aga ttg cct caa    735
   Ala Asn Arg Leu Phe Phe Asp Thr Ser Asn Gln Ser Arg Leu Pro Gln
           125                 130                 135
   ttt gag att gca gga tat gaa gga gta tcc att tca ctt ttt act cag    783
   Phe Glu Ile Ala Gly Tyr Glu Gly Val Ser Ile Ser Leu Phe Thr Gln
       140                 145                 150
   atg tgt aca ttt cat ttg ggt tta tta aaa gat gga att tta gca gga    831
   Met Cys Thr Phe His Leu Gly Leu Leu Lys Asp Gly Ile Leu Ala Gly
       155                 160                 165
   agc gat tgg gga ttt gct cct gca gat aaa gac gct ctt att tgc caa    879
   Ser Asp Trp Gly Phe Ala Pro Ala Asp Lys Asp Ala Leu Ile Cys Gln
   170                 175                 180                 185
   ttc aat aga ttt gtc aat gaa tat aat act cga ctg atg gta ttg tac    927
   Phe Asn Arg Phe Val Asn Glu Tyr Asn Thr Arg Leu Met Val Leu Tyr
                   190                 195                 200
   tca aaa gaa ttt gga cgg tta tta gca aaa aat ctt aat gaa gcc ttg    975
   Ser Lys Glu Phe Gly Arg Leu Leu Ala Lys Asn Leu Asn Glu Ala Leu
           205                 210                 215
   aac ttt aga aat atg tgt agt tta tat gtc ttt cct ttt tct gaa gca    1023
   Asn Phe Arg Asn Met Cys Ser Leu Tyr Val Phe Pro Phe Ser Glu Ala
       220                 225                 230
   tgg tct tta tta agg tat gaa gga aca aaa tta gaa aac acg ctt tca    1071
   Trp Ser Leu Leu Arg Tyr Glu Gly Thr Lys Leu Glu Asn Thr Leu Ser
       235                 240                 245
   tta tgg aat ttt gtg ggt gaa agt atc aat aat ata tct cct aat gat    1119
   Leu Trp Asn Phe Val Gly Glu Ser Ile Asn Asn Ile Ser Pro Asn Asp
   250                 255                 260                 265
   tgg aaa ggt gcg ctt tat aaa ttg tta atg gga gca cct aat caa aga    1167
   Trp Lys Gly Ala Leu Tyr Lys Leu Leu Met Gly Ala Pro Asn Gln Arg
                   270                 275                 280
   tta aac aat gtt aag ttt aat tat agt tat ttt tct gat act caa gcg    1215
   Leu Asn Asn Val Lys Phe Asn Tyr Ser Tyr Phe Ser Asp Thr Gln Ala
           285                 290                 295
   aca ata cat cgt gaa aac att cat ggt gtc ctg cca aca                      1254
   Thr Ile His Arg Glu Asn Ile His Gly Val Leu Pro Thr
           300                 305                 310
<210> 9
   <211> 310
   <212> PRT
   <213> Bacillus thuringiensis
<400> 9
   Met Gln Asn Asn Asn Phe Asn Thr Thr Glu Ile Asn Asn Met Ile Asn
   1 5 10 15
   Phe Pro Met Tyr Asn Gly Arg Leu Glu Pro Ser Leu Ala Pro Ala Leu
   20 25 30
   Ile Ala Val Ala Pro Ile Ala Lys Tyr Leu Ala Thr Ala Leu Ala Lys
   35 40 45
   Trp Ala Val Lys Gln Gly Phe Ala Lys Leu Lys Ser Glu Ile Phe Pro
   50 55 60
   Gly Asn Thr Pro Ala Thr Met Asp Lys Val Arg Ile Glu Val Gln Thr
   65 70 75 80
   Leu Leu Asp Gln Arg Leu Gln Asp Asp Arg Val Lys Ile Leu Glu Gly
   85 90 95
   Glu Tyr Lys Gly Ile Ile Asp Val Ser Lys Val Phe Thr Asp Tyr Val
   100 105 110
   Asn Gln Ser Lys Phe Glu Thr Gly Thr Ala Asn Arg Leu Phe Phe Asp
   115 120 125
   Thr Ser Asn Gln Ser Arg Leu Pro Gln Phe Glu Ile Ala Gly Tyr Glu
   130 135 140
   Gly Val Ser Ile Ser Leu Phe Thr Gln Met Cys Thr Phe His Leu Gly
   145 150 155 160
   Leu Leu Lys Asp Gly Ile Leu Ala Gly Ser Asp Trp Gly Phe Ala Pro
   165 170 175
   Ala Asp Lys Asp Ala Leu Ile Cys Gln Phe Asn Arg Phe Val Asn Glu
   180 185 190
   Tyr Asn Thr Arg Leu Met Val Leu Tyr Ser Lys Glu Phe Gly Arg Leu
           195                 200                 205
   Leu Ala Lys Asn Leu Asn Glu Ala Leu Asn Phe Arg Asn Met Cys Ser
       210                 215                 220
   Leu Tyr Val Phe Pro Phe Ser Glu Ala Trp Ser Leu Leu Arg Tyr Glu
   225                 230                 235                 240
   Gly Thr Lys Leu Glu Asn Thr Leu Ser Leu Trp Asn Phe Val Gly Glu
                   245                 250                 255
   Ser Ile Asn Asn Ile Ser Pro Asn Asp Trp Lys Gly Ala Leu Tyr Lys
               260                 265                 270
   Leu Leu Met Gly Ala Pro Asn Gln Arg Leu Asn Asn Val Lys Phe Asn
           275                 280                 285
   Tyr Ser Tyr Phe Ser Asp Thr Gln Ala Thr Ile His Arg Glu Asn Ile
       290                 295                 300
   His Gly Val Leu Pro Thr
   305                 310
<210> 10
   <211> 2434
   <212> ADN
   <213> Bacillus thuringiensis
<400> 10
   ttaattatga aaagatttcg tttatattag taaattgttt aaagaagagg gggcatgttt 60
   taaatgcaaa ataacaactt taataccaca gaaattaata atatgattaa tttccctatg 120
   tataatggta gattagaacc ttctctagct ccagcattaa tagcagtagc tccaattgct 180
   aaatatttag caacagctct tgctaaatgg gctgtaaaac aagggtttgc aaaattaaaa 240
   tccgagatat tccccggtaa tacgcctgct actatggata aggttcgtat tgaggtacaa 300
   acacttttag accaaagatt acaagatgac agagttaaga ttttagaagg tgaatacaaa 360
   ggaattattg acgtgagtaa agtttttact gattatgtta atcaatctaa atttgagact 420
   ggaacagcta ataggctttt ttttgataca agtaaccaat taataagcag attgcctcaa 480
   tttgagattg caggatatga aggagtatcc atttcacttt ttactcagat gtgtacattt 540
   catttgggtt tattaaaaga tggaatttta gcaggaagcg attggggatt tgctcctgca 600
   gataaagacg ctcttatttg ccaattcaat agatttgtca atgaatataa tactcgactg 660
   atggtattgt actcaaaaga atttggacgg ttattagcaa aaaatcttaa tgaagccttg 720
   aactttagaa atatgtgtag tttatatgtc tttccttttt ctgaagcatg gtctttatta 780
   aggtatgaag gaacaaaatt agaaaacacg ctttcattat ggaattttgt gggtgaaagt 840
   atcaataata tatctcctaa tgattggaaa ggtgcgcttt ataaattgtt aatgggagca 900
   cctaatcaaa gattaaacaa tgttaagttt aattatagtt atttttctga tactcaagcg 960
   acaatacatc gtgaaaacat tcatggtgtc ctgccaacat ataatggagg accaacaatt 1020
   acaggatgga tagggaatgg gcgtttcagc ggacttagtt ttccttgtag taatgaatta 1080
   gaaattacaa aaataaaaca ggaaataact tacaatgata aagggggaaa tttcaattca 1140
   atagttcctg ctgctacgcg caatgaaatt ctaactgcta ccgttccaac atcagctgat 1200
   ccatttttta aaaccgctga tattaactgg aaatatttct ctccgggtct ttactctgga 1260
   tggaatatta aatttgatga tacagtcact ttaaaaagta gagtaccaag tattatacct 1320
   tcaaatatat taaagtatga tgattattat attcgtgccg tttcagcctg tccaaaaggc 1380
   gtatcacttg catataacca tgatttttta acgttaacat ataataaatt agaatatgat 1440
   gcacctacta cacaaaatat cattgtagga ttttcaccag ataatactaa gagtttttat 1500
   aggagcaact ctcattatct aagtacaaca gatgatgcct atgtaattcc tgctttacaa 1560
   ttttctacag tctcagatag atcattctta gaagatacac cagatcaagc aacagatggc 1620
   agtattaaat ttacggatac tgttcttggg aatgaggcaa aatattctat tagactaaat 1680
   actggattta atacagctac taggtataga ttaattatac gttttaaagc gcctgctcgt 1740
   ttggctgctg gtatacgtgt acgttctcaa aattcaggga ataataagtt attaggtggt 1800
   attcctgtag agggtaattc tggatggata gattatatta cagattcatt tacttttgat 1860
   gaccttggga ttacaacttc aagtacaaat gctttcttta gtattgattc agatggtgta 1920
   aatgcttctc aacaatggta tttgtctaaa ttaattttag taaaagaatc cagttttacg 1980
   actcagattc cattaaaacc atacgttatt gtacgttgtc cggatacttt ttttgtgagc 2040
   aacaattcaa gtagtacgta cgaacaaggc tataacaaca attacaacca gaattctagc 2100
   agtatgtacg atcaaggcta taacaatagc tataatccaa actctggttg tacgtgtaat 2160
   caagactata acaatagcta taaccaaaac tctggctgta catgtaacca agggtataac 2220
   aataactatc ctaaataaga aaacaatgaa aaagcattcc cctctcacaa ggaatgcttt 2280
   tttgtctgcc ctattttacg catatataaa acccattggt aattgcatac tatgcatact 2340
   ctataaaacc gttccatcct acccctgtta tgaagtgacc tttgtcaata gtttttcaac 2400
   cataatattt tttcttgatg gcatacaaaa gctt                             2434
<210> 11
   <211> 3675
   <212> ADN
   <213> Bacillus thuringiensis
<400> 11
   aagtgatcct tctcaagtaa cggttaatct ttctgaagta aaatatataa ggcatgaggt 60
   tctgttgcaa agtttgaaaa aagtataaac gccttggaaa atgatgagaa atagttaagc 120
   agtcgctagt aattgttgca gttcattgta cgcagaaaag acggagtctg attcaaactt 180
   cgtctctgtt tatataaggc ctgaatcgtt tcaatccctt ttatggtacg tgaagcatgg 240
   cggatatttt ggaatccaga agatttggca aaacgtcgtt ttacatgtcg gtggtcttgt 300
   tcaatcaggt tattaagatg tttaaccgta caatgttttg tatgcacata cacaccgttc 360
   ttttttagtt ttttaaacgc acaaagcagt gcaggagcct tatctgtaat aagaaccttt 420
   ggttctccaa aagttttcac taatcgtttc ataaacatat aagcggcttg atgatcgcgt 480
   gtttgacgaa gttgaaaatc aagcgtatgt ccatcactat caatagcacg atatagatag 540
   caccattcgc ctttgacttt gatatacgtt tcatctaaat gccatgacga ttgagatgtt 600
   gtgtttttct ttttccagat ttgatagatc agattgccat attcatgcac ccatcgcatg 660
   attgttgttg ggtgaacgga aacgcctcgt tctttcagaa tttcagatac atcacgatag 720
   cttaaagaaa aacgacaata gtagccaacg gctacaaaaa taatatcttt cttgaattgt 780
   tttcctttaa aatatcccat ttgcaattct cctcacagct ttttctaaat tgtaaccagc 840
   tgaaaagaac tttgcaacag aaccctttgg taatactctg cgtaaaaaag gattgaccag 900
   gtgtgacttt gaagtgtata caaatcccgc atatgcaggt ttttttgttt ctggtgcaaa 960
   aagataacaa attaaaaaaa caggaaataa actaacgatt gacgaaatag aagtgaaaat 1020
   agaaaaggat gtgaggatta ttacgtgaat actgattatg ataattttga aaagaaatca 1080
   tatttgcaac cagaatcatc atatgattac atgtcagaag aaaatcacaa agttccatta 1140
   attagatgtg aattcctatt tttatttcag tccagaatcc tgaataatgg aaattaaatg 1200
   caccctatga tttataaata tatgtacctt taaaacaaga ataattataa ctgtataaaa 1260
   ataggtatac tattggaaaa caaaaaagtt aattatgaaa agatttcgtt tatattagta 1320
   aattgtttaa agaagagggg gcatgtttta aatgcaaaat aacaacttta ataccacaga 1380
   aattaataat atgattaatt tccctatgta taatggtaga ttagaacctt ctctagctcc 1440
   agcattaata gcagtagctc caattgctaa atatttagca acagctcttg ctaaatgggc 1500
   tgtaaaacaa gggtttgcaa aattaaaatc cgagatattc cccggtaata cgcctgctac 1560
   tatggataag gttcgtattg aggtacaaac acttttagac caaagattac aagatgacag 1620
   agttaagatt ttagaaggtg aatacaaagg aattattgac gtgagtaaag tttttactga 1680
   ttatgttaat caatctaaat ttgagactgg aacagctaat aggctttttt ttgatacaag 1740
   taaccaatta ataagcagat tgcctcaatt tgagattgca ggatatgaag gagtatccat 1800
   ttcacttttt actcagatgt gtacatttca tttgggttta ttaaaagatg gaattttagc 1860
   aggaagcgat tggggatttg ctcctgcaga taaagacgct cttatttgcc aattcaatag 1920
   atttgtcaat gaatataata ctcgactgat ggtattgtac tcaaaagaat ttggacggtt 1980
   attagcaaaa aatcttaatg aagccttgaa ctttagaaat atgtgtagtt tatatgtctt 2040
   tcctttttct gaagcatggt ctttattaag gtatgaagga acaaaattag aaaacacgct 2100
   ttcattatgg aattttgtgg gtgaaagtat caataatata tctcctaatg attggaaagg 2160
   tgcgctttat aaattgttaa tgggagcacc taatcaaaga ttaaacaatg ttaagtttaa 2220
   ttatagttat ttttctgata ctcaagcgac aatacatcgt gaaaacattc atggtgtcct 2280
   gccaacatat aatggaggac caacaattac aggatggata gggaatgggc gtttcagcgg 2340
   acttagtttt ccttgtagta atgaattaga aattacaaaa ataaaacagg aaataactta 2400
   caatgataaa gggggaaatt tcaattcaat agttcctgct gctacgcgca atgaaattct 2460
   aactgctacc gttccaacat cagctgatcc attttttaaa accgctgata ttaactggaa 2520
   atatttctct ccgggtcttt actctggatg gaatattaaa tttgatgata cagtcacttt 2580
   aaaaagtaga gtaccaagta ttataccttc aaatatatta aagtatgatg attattatat 2640
   tcgtgccgtt tcagcctgtc caaaaggcgt atcacttgca tataaccatg attttttaac 2700
   gttaacatat aataaattag aatatgatgc acctactaca caaaatatca ttgtaggatt 2760
   ttcaccagat aatactaaga gtttttatag gagcaactct cattatctaa gtacaacaga 2820
   tgatgcctat gtaattcctg ctttacaatt ttctacagtc tcagatagat cattcttaga 2880
   agatacacca gatcaagcaa cagatggcag tattaaattt acggatactg ttcttgggaa 2940
   tgaggcaaaa tattctatta gactaaatac tggatttaat acagctacta ggtatagatt 3000
   aattatacgt tttaaagcgc ctgctcgttt ggctgctggt atacgtgtac gttctcaaaa 3060
   ttcagggaat aataagttat taggtggtat tcctgtagag ggtaattctg gatggataga 3120
   ttatattaca gattcattta cttttgatga ccttgggatt acaacttcaa gtacaaatgc 3180
   tttctttagt attgattcag atggtgtaaa tgcttctcaa caatggtatt tgtctaaatt 3240
   aattttagta aaagaatcca gttttacgac tcagattcca ttaaaaccat acgttattgt 3300
   acgttgtccg gatacttttt ttgtgagcaa caattcaagt agtacgtacg aacaaggcta 3360
   taacaacaat tacaaccaga attctagcag tatgtacgat caaggctata acaatagcta 3420
   taatccaaac tctggttgta cgtgtaatca agactataac aatagctata accaaaactc 3480
   tggctgtaca tgtaaccaag ggtataacaa taactatcct aaataagaaa acaatgaaaa 3540
   agcattcccc tctcacaagg aatgcttttt tgtctgccct attttacgca tatataaaac 3600
   ccattggtaa ttgcatacta tgcatactct ataaaaccgt tccatcctac ccctgttatg 3660
   aagtgacctt tgtca                                                  3675
<210> 12
   <211> 724
   <212> PRT
   <213> Bacillus thuringiensis
<400> 12
   Met Gln Asn Asn Asn Phe Asn Thr Thr Glu Ile Asn Asn Met Ile Asn
    1               5                  10                  15
   Phe Pro Met Tyr Asn Gly Arg Leu Glu Pro Ser Leu Ala Pro Ala Leu
               20                  25                  30
   Ile Ala Val Ala Pro Ile Ala Lys Tyr Leu Ala Thr Ala Leu Ala Lys
           35                  40                  45
   Trp Ala Val Lys Gln Gly Phe Ala Lys Leu Lys Ser Glu Ile Phe Pro
       50                  55                  60
   Gly Asn Thr Pro Ala Thr Met Asp Lys Val Arg Ile Glu Val Gln Thr
   65                  70                  75                  80
   Leu Leu Asp Gln Arg Leu Gln Asp Asp Arg Val Lys Ile Leu Glu Gly
                   85                  90                  95
   Glu Tyr Lys Gly Ile Ile Asp Val Ser Lys Val Phe Thr Asp Tyr Val
               100                 105                 110
   Asn Gln Ser Lys Phe Glu Thr Gly Thr Ala Asn Arg Leu Phe Phe Asp
           115                 120                 125
   Thr Ser Asn Gln Leu Ile Ser Arg Leu Pro Gln Phe Glu Ile Ala Gly
       130                 135                 140
   Tyr Glu Gly Val Ser Ile Ser Leu Phe Thr Gln Met Cys Thr Phe His
   145                 150                 155                 160
   Leu Gly Leu Leu Lys Asp Gly Ile Leu Ala Gly Ser Asp Trp Gly Phe
                   165                 170                 175
   Ala Pro Ala Asp Lys Asp Ala Leu Ile Cys Gln Phe Asn Arg Phe Val
               180                 185                 190
   Asn Glu Tyr Asn Thr Arg Leu Met Val Leu Tyr Ser Lys Glu Phe Gly
           195                 200                 205
   Arg Leu Leu Ala Lys Asn Leu Asn Glu Ala Leu Asn Phe Arg Asn Met
       210                 215                 220
   Cys Ser Leu Tyr Val Phe Pro Phe Ser Glu Ala Trp Ser Leu Leu Arg
   225                 230                 235                 240
   Tyr Glu Gly Thr Lys Leu Glu Asn Thr Leu Ser Leu Trp Asn Phe Val
                   245                 250                 255
   Gly Glu Ser Ile Asn Asn Ile Ser Pro Asn Asp Trp Lys Gly Ala Leu
               260                 265                 270
   Tyr Lys Leu Leu Met Gly Ala Pro Asn Gln Arg Leu Asn Asn Val Lys
           275                 280                 285
   Phe Asn Tyr Ser Tyr Phe Ser Asp Thr Gln Ala Thr Ile His Arg Glu
       290                 295                 300
   Asn Ile His Gly Val Leu Pro Thr Tyr Asn Gly Gly Pro Thr Ile Thr
   305                 310                 315                 320
   Gly Trp Ile Gly Asn Gly Arg Phe Ser Gly Leu Ser Phe Pro Cys Ser
                   325                 330                 335
   Asn Glu Leu Glu Ile Thr Lys Ile Lys Gln Glu Ile Thr Tyr Asn Asp
               340                 345                 350
   Lys Gly Gly Asn Phe Asn Ser Ile Val Pro Ala Ala Thr Arg Asn Glu
           355                 360                 365
   Ile Leu Thr Ala Thr Val Pro Thr Ser Ala Asp Pro Phe Phe Lys Thr
       370                 375                 380
   Ala Asp Ile Asn Trp Lys Tyr Phe Ser Pro Gly Leu Tyr Ser Gly Trp
   385                 390                 395                 400
   Asn Ile Lys Phe Asp Asp Thr Val Thr Leu Lys Ser Arg Val Pro Ser
                   405                 410                 415
   Ile Ile Pro Ser Asn Ile Leu Lys Tyr Asp Asp Tyr Tyr Ile Arg Ala
               420                 425                 430
   Val Ser Ala Cys Pro Lys Gly Val Ser Leu Ala Tyr Asn His Asp Phe
           435                 440                 445
   Leu Thr Leu Thr Tyr Asn Lys Leu Glu Tyr Asp Ala Pro Thr Thr Gln
       450                 455                 460
   Asn Ile Ile Val Gly Phe Ser Pro Asp Asn Thr Lys Ser Phe Tyr Arg
   465                 470                 475                 480
   Ser Asn Ser His Tyr Leu Ser Thr Thr Asp Asp Ala Tyr Val Ile Pro
                   485                 490                 495
   Ala Leu Gln Phe Ser Thr Val Ser Asp Arg Ser Phe Leu Glu Asp Thr
               500                 505                 510
   Pro Asp Gln Ala Thr Asp Gly Ser Ile Lys Phe Thr Asp Thr Val Leu
           515                 520                 525
   Gly Asn Glu Ala Lys Tyr Ser Ile Arg Leu Asn Thr Gly Phe Asn Thr
       530                 535                 540
   Ala Thr Arg Tyr Arg Leu Ile Ile Arg Phe Lys Ala Pro Ala Arg Leu
   545                 550                 555                 560
   Ala Ala Gly Ile Arg Val Arg Ser Gln Asn Ser Gly Asn Asn Lys Leu
                   565                 570                 575
   Leu Gly Gly Ile Pro Val Glu Gly Asn Ser Gly Trp Ile Asp Tyr Ile
               580                 585                 590
   Thr Asp Ser Phe Thr Phe Asp Asp Leu Gly Ile Thr Thr Ser Ser Thr
           595                 600                 605
   Asn Ala Phe Phe Ser Ile Asp Ser Asp Gly Val Asn Ala Ser Gln Gln
       610                 615                 620
   Trp Tyr Leu Ser Lys Leu Ile Leu Val Lys Glu Ser Ser Phe Thr Thr
   625                 630                 635                 640
   Gln Ile Pro Leu Lys Pro Tyr Val Ile Val Arg Cys Pro Asp Thr Phe
                   645                 650                 655
   Phe Val Ser Asn Asn Ser Ser Ser Thr Tyr Glu Gln Gly Tyr Asn Asn
               660                 665                 670
   Asn Tyr Asn Gln Asn Ser Ser Ser Met Tyr Asp Gln Gly Tyr Asn Asn
           675                 680                 685
   Ser Tyr Asn Pro Asn Ser Gly Cys Thr Cys Asn Gln Asp Tyr Asn Asn
       690                 695                 700
   Ser Tyr Asn Gln Asn Ser Gly Cys Thr Cys Asn Gln Gly Tyr Asn Asn
   705                 710                 715                 720
   Asn Tyr Pro Lys
<210> 13
   <211> 643
   <212> PRT
   <213> Bacillus thuringiensis
<400> 13
   Met Glu Asp Ser Ser Leu Asp Thr Leu Ser Ile Val Asn Glu Thr Asp
    1               5                  10                  15
   Phe Pro Leu Tyr Asn Asn Tyr Thr Glu Pro Thr Ile Ala Pro Ala Leu
               20                  25                  30
   Ile Ala Val Ala Pro Ile Ala Gln Tyr Leu Ala Thr Ala Ile Gly Lys
           35                  40                  45
   Trp Ala Ala Lys Ala Ala Phe Ser Lys Val Leu Ser Leu Ile Phe Pro
       50                  55                  60
   Gly Ser Gln Pro Ala Thr Met Glu Lys Val Arg Thr Glu Val Glu Thr
   65                  70                  75                  80
   Leu Ile Asn Gln Lys Leu Ser Gln Asp Arg Val Asn Ile Leu Asn Ala
                   85                  90                  95
   Glu Tyr Arg Gly Ile Ile Glu Val Ser Asp Val Phe Asp Ala Tyr Ile
               100                 105                 110
   Lys Gln Pro Gly Phe Thr Pro Ala Thr Ala Lys Gly Tyr Phe Leu Asn
           115                 120                 125
   Leu Ser Gly Ala Ile Ile Gln Arg Leu Pro Gln Phe Glu Val Gln Thr
       130                 135                 140
   Tyr Glu Gly Val Ser Ile Ala Leu Phe Thr Gln Met Cys Thr Leu His
   145                 150                 155                 160
   Leu Thr Leu Leu Lys Asp Gly Ile Leu Ala Gly Ser Ala Trp Gly Phe
                   165                 170                 175
   Thr Gln Ala Asp Val Asp Ser Phe Ile Lys Leu Phe Asn Gln Lys Val
               180                 185                 190
   Leu Asp Tyr Arg Thr Arg Leu Met Arg Met Tyr Thr Glu Glu Phe Gly
           195                 200                 205
   Arg Leu Cys Lys Val Ser Leu Lys Asp Gly Leu Thr Phe Arg Asn Met
       210                 215                 220
   Cys Asn Leu Tyr Val Phe Pro Phe Ala Glu Ala Trp Ser Leu Met Arg
   225                 230                 235                 240
   Tyr Glu Gly Leu Lys Leu Gln Ser Ser Leu Ser Leu Trp Asp Tyr Val
                   245                 250                 255
   Gly Val Ser Ile Pro Val Asn Tyr Asn Glu Trp Gly Gly Leu Val Tyr
               260                 265                 270
   Lys Leu Leu Met Gly Glu Val Asn Gln Arg Leu Thr Thr Val Lys Phe
           275                 280                 285
   Asn Tyr Ser Phe Thr Asn Glu Pro Ala Asp Ile Pro Ala Arg Glu Asn
       290                 295                 300
   Ile Arg Gly Val His Pro Ile Tyr Asp Pro Ser Ser Gly Leu Thr Gly
   305                 310                 315                 320
   Trp Ile Gly Asn Gly Arg Thr Asn Asn Phe Asn Phe Ala Asp Asn Asn
                   325                 330                 335
   Gly Asn Glu Ile Met Glu Val Arg Thr Gln Thr Phe Tyr Gln Asn Pro
               340                 345                 350
   Asn Asn Glu Pro Ile Ala Pro Arg Asp Ile Ile Asn Gln Ile Leu Thr
           355                 360                 365
   Ala Pro Ala Pro Ala Asp Leu Phe Phe Lys Asn Ala Asp Ile Asn Val
       370                 375                 380
   Lys Phe Thr Gln Trp Phe Gln Ser Thr Leu Tyr Gly Trp Asn Ile Lys
   385                 390                 395                 400
   Leu Gly Thr Gln Thr Val Leu Ser Ser Arg Thr Gly Thr Ile Pro Pro
                   405                 410                 415
   Asn Tyr Leu Ala Tyr Asp Gly Tyr Tyr Ile Arg Ala Ile Ser Ala Cys
               420                 425                 430
   Pro Arg Gly Val Ser Leu Ala Tyr Asn His Asp Leu Thr Thr Leu Thr
           435                 440                 445
   Tyr Asn Arg Ile Glu Tyr Asp Ser Pro Thr Thr Glu Asn Ile Ile Val
       450                 455                 460
   Gly Phe Ala Pro Asp Asn Thr Lys Asp Phe Tyr Ser Lys Lys Ser His
   465                 470                 475                 480
   Tyr Leu Ser Glu Thr Asn Asp Ser Tyr Val Ile Pro Ala Leu Gln Phe
                   485                 490                 495
   Ala Glu Val Ser Asp Arg Ser Phe Leu Glu Asp Thr Pro Asp Gln Ala
               500                 505                 510
   Thr Asp Gly Ser Ile Lys Phe Ala Arg Thr Phe Ile Ser Asn Glu Ala
           515                 520                 525
   Lys Tyr Ser Ile Arg Leu Asn Thr Gly Phe Asn Thr Ala Thr Arg Tyr
       530                 535                 540
   Lys Leu Ile Ile Arg Val Arg Val Pro Tyr Arg Leu Pro Ala Gly Ile
   545                 550                 555                 560
   Arg Val Gln Ser Gln Asn Ser Gly Asn Asn Arg Met Leu Gly Ser Phe
                   565                 570                 575
   Thr Ala Asn Ala Asn Pro Glu Trp Val Asp Phe Val Thr Asp Ala Phe
               580                 585                 590
   Thr Phe Asn Asp Leu Gly Ile Thr Thr Ser Ser Thr Asn Ala Leu Phe
           595                 600                 605
   Ser Ile Ser Ser Asp Ser Leu Asn Ser Gly Glu Glu Trp Tyr Leu Ser
       610                 615                 620
   Gln Leu Phe Leu Val Lys Glu Ser Ala Phe Thr Thr Gln Ile Asn Pro
   625                 630                 635                 640
   Leu Leu Lys